# EUROPEAN PATENT APPLICATION

(11) **EP 4 707 296 A1**
(43) Date of publication of application: **11.03.2026**
(21) Application number: 24306450.8
(22) Date of filing: 05.09.2024
(51) Int. Cl.: C07K 14/55

(54) **INTERLEUKIN-2 VARIANTS WITH MODIFIED BIOLOGICAL ACTIVITY**

(71) Applicant: Egle Therapeutics, 75014 Paris (FR)
(72) Inventor: The designation of the inventor has not yet been filed
(74) Representative: Ipsilon

(57) **Abstract**

The present invention relates to interleukin-2 variants, to pharmaceutical compositions comprising said interleukin-2 variants, in a pharmaceutically acceptable carrier, and to their implementation in the prevention or treatment of cancer, or in the prevention or treatment of a disease selected from the group consisting of acute or chronic inflammatory diseases, allergic diseases, autoimmune diseases, graft-versus-host disease and graft rejection.

## Description

### FIELD OF THE INVENTION

The invention relates to Interleukin-2 (IL-2) variants for the prevention or treatment of immune disorders, such as for example acute or chronic inflammatory, allergic, autoimmune or infectious diseases, graft-versus-host disease, graft-rejection, and cancer.

### BACKGROUND OF THE INVENTION

Interleukin-2 (IL-2) is a key cytokine of the immune response which promotes the activation, proliferation and survival of T and B lymphocytes (T and B cells). IL-2 receptor (IL-2R) is a heterotrimeric protein complex composed of an alpha (IL-2-RA or CD25), a beta (IL-2RB or CD122) and a gamma (IL-2RG or Gamma c or CD132) chain. The alpha chain binds IL-2 with low affinity and does not participate in signaling. The combination of the beta and gamma chains forms an intermediate affinity receptor expressed on the effector T cells (comprising conventional T cells (Tconv; CD4+Foxp3-) and CD8+ Tcells) and NK cells responsible for the cell-mediated immune responses; and all three receptor chains form a high affinity receptor expressed constitutively on regulatory T cells (Tregs; CD4+Foxp3+) which have immunosuppressive function, and shortly or transiently by activated effector T cells and NK cells.

Indeed, IL-2 is a cytokine with both immune stimulating and suppressive function. Consequently, it has been used in the clinic at high doses to stimulate the immune response against cancer, and more recently at low doses to block the immune response in various physiopathological conditions including autoimmune diseases such as type 1 diabetes (T1D), autoimmune vasculitis, inflammatory diseases such as Parkinson disease and parasitic infection such as Trypanosoma cruzi infection; and graft rejection including graft-versus-host disease (GVHD) (Rosenberg et al, Sci. Transl. Med., 2012, 4, 127-; Grinberg-Bleyer, Y. et al, J. Exp. Med., 2010, 207, 1871-1878; Tang et al, Immunity, 2008, 28, 687-697; Pilon et al, Am. J. Transplant., 2014, 14, 2874-2882; Saadoun et al, N. Engl. J. Med., 2011, 365, 2067-2077; Koreth et al, N. Engl. J. Med., 2011, 365, 2055-2066; Kennedy-Nasser et al., Clin. Cancer Res. Off. J. Am. Assoc. Cancer Res., 2014, 20, 2215-2225; Baeyens et al., Diabetes, 2013, 62, 3120-3131; Gonzalez et al., Brain. Behav. Immun., 2015, 45, 219-232; Perol et al., Immunol. Lett., 2014, 162, 173-184).

However, high-doses of IL-2 in cancer therapy can be very toxic and its efficacy is not optimal (5-20 % of responders) due to the unwanted effect of IL-2 on Tregs.

In humans, low doses of IL-2 have been successfully administered to boost Tregs and dampen inflammation in autoimmune vasculitis patients and in graft-versus-host disease (Saadoun et al, N. Engl. J. Med., 2011, 365, 2067-2077; Koreth et al, N. Engl. J. Med., 2011, 365, 2055-2066; Kennedy-Nasser et al., Clin. Cancer Res. Off. J. Am. Assoc. Cancer Res., 2014, 20, 2215-2225). However, improvements need to be done as IL-2 has a very short half-life and needs repeated administration.

Interestingly, when IL-2 is complexed with an anti-IL-2 antibody (Ab), IL-2 pharmacodynamics is improved, its toxicity is lowered, and depending on the Ab, this complex can re-direct IL-2 action to effector (pro-Teff anti-IL-2 Ab) or to regulatory (pro-Treg anti-IL-2 Ab) immune cells, solving the main problems associated with IL-2-based therapies (Boyman et al., Science, 2006, 311, 1924-1927. Letourneau et al., Proc. Natl. Acad. Sci., 2010, 107, 2171-2176). Concerning the mechanism of action, it was shown that the formation of a complex with pro-Teff anti-IL-2 antibody blocks directly the interaction of IL-2 with CD25 (Levin et al., Nature, 2012, 484, 529-533; Rojas, G. in Monoclonal Antibodies (eds. Ossipow, V. & Fischer, N.) 1131, 447-476 (Humana Press, 2014)).

Treg-directed IL-2/anti-IL-2 complexes gave impressive results in different mouse models of inflammation: T1D, asthma, EAE, atherosclerosis, chronic nephropathy and transplantation, including solid organ transplantation (Webster et al., J. Exp. Med., 2009, 206, 751-760; Dinh, T. N. et al., Circulation, 2012, 126, 1256-1266; Polhill, T. et al. J. Am. Soc. Nephrol. JASN, 2012, 23, 1303-1308; Satake et al., PLoS ONE, 2014, 9, e92888 ; Vokaer et al., Transplant. Proc., 2012, 44, 2840-2844; Goldstein et al., Front. Immunol., 2013, 4, 155).

Another strategy to fine-tune IL-2 biological activity consists in performing punctual mutations in the IL-2 molecule to generate an IL-2 variant or mutant IL-2. A mutant IL-2 protein (IL-2 superkine) comprising five punctual mutations which confer a 300-fold higher affinity for CD 122 compared to wild type IL-2 has been disclosed (Levin et al., Nature, 2012, 484, 529-533). Immunosuppressive IL-2 variants with greater affinity for IL-2RA and/or altered signaling through IL-2RBG have also been disclosed (WO 2010/085495 and US 2014/0286898).

To improve IL-2 based immunotherapy, there is a need for IL-2 variants for the prevention or treatment of immune disorders.

To improve cancer treatment, there is a need for new actives and therapies aiming at selectively depleting tumor infiltrating Tregs.

There is furthermore a need for a robust new antitumor agent which can directly or indirectly enhance the immune system's ability to recognize and destroy cancer cells.

There is a need for innovative strategies to overcome the limitations of current cancer immunotherapies by addressing specific challenges such as Tregs-mediated immunosuppression and improving the specificity and efficacy of therapeutic antitumoral interventions.

The present invention aims to address these challenges by providing new compounds, in the form of new IL-2 variants.

### SUMMARY OF THE INVENTION

The inventors have engineered variants of human IL-2 which are capable of preferentially stimulating (IL-2 agonists) or antagonizing (IL-2 antagonists) IL-2 signaling in Tregs.

The examples of the present application show variants of human IL-2 (Treg-agonist variants) which selectively activate STATS phosphorylation (i.e. IL-2 signaling) in Tregs *in vitro.* The Treg-agonist variants are useful for reducing immune activation indirectly, by preferentially activating Tregs that in turn inhibit the immune effector cells. The Treg-agonist variants of the invention are thus useful for treating diseases where immunomodulation or immunosuppression is beneficial such as with no limitations, allergic and autoimmune diseases, and diseases comprising overactivity of the immune system including in particular, chronic or acute inflammatory diseases, graft-versus-host disease (GVHD) and graft rejection.

The examples of the present application also show that some of the variants of human IL-2 according to the invention are IL-2 antagonists. These variants inhibit IL-2 induced STAT-5 phosphorylation in Tregs and therefore reduce Treg proliferation and survival *in vitro* by depriving them from wild-type IL-2 signaling (similar to a blocking anti-IL-2 antibody). *In vivo,* it is expected that inhibition of Tregs with the IL-2 antagonist variants of the invention will reduce tumor growth as previously shown with other IL-2 antagonists (Carmenate et al., The Journal of Immunology, 2018, 200, 3475-3484). Treg inhibition by the IL-2 antagonist variants according to the invention should consequently promote immune responses by unleashing immune cells, such as lymphocytes (B, NK, CD4+ or CD8+ T cells), dendritic cells (DC), macrophages and others, from Treg suppression. In addition, CD8+ T cell function should be preserved or moderately impacted, depending on the used dose of IL-2 antagonist variant. Therefore, the IL-2 antagonist variants should further allow the direct stimulation of a CD8+ T cell immune response, for example against a tumor, a pathogen or a vaccine. For all these reasons, better immune responses to cancer, infectious agents and vaccines are expected with the IL-2-antagonist variants of the invention.

The results present in the herein examples show the potential of these human IL-2 variants for the treatment of immune disorders and for increasing immune responses against vaccines. Immune disorders include in particular inflammatory, allergic, infectious and autoimmune diseases, graft-versus-host disease (GVHD), graft rejection, and cancer. Vaccines may be directed to cancer or infectious diseases.

The variants of IL-2 according to the invention can also be used to screen for anti-IL-2 antibodies with pro-Teff or pro-Treg activity.

Therefore, the invention relates to interleukin-2 (IL-2) variants capable of preferentially stimulating or antagonizing IL-2 in Tregs.

A first object of the present invention relates to an interleukin-2 variant which comprises at least:
(i) the amino acid substitution H16R; or
(ii) the amino acid substitution H16R and one or two amino acid substitution(s) selected from the group consisting of L19R and V91K; or
(iii) the amino acid substitution L19R and the amino acid substitution V91K;

the identified positions being determined by alignment with the sequence set forth as SEQ ID NO: 1,
the IL-2 variant comprising at most 10 substitutions compared with sequence SEQ ID NO: 1.

In particular, an interleukin-2 variant may be selected from the group consisting of:
- a variant comprising the amino acid substitution H16R; or
- a variant comprising the amino acid substitutions H16R and L19R; or
- a variant comprising the amino acid substitutions H16R and V91K; or
- a variant comprising the amino acid substitutions L19R and V91K; or
- a variant comprising the amino acid substitutions H16R, L19R and V91K;
the identified positions being determined by alignment with the sequence set forth as SEQ ID NO: 1.

An interleukin-2 variant according to the invention may in particular be characterized in that it:
- does not comprise any substitution at at least one of the positions selected from the group consisting of positions 9, 12, 23, 26, 31, 87 and 95, and in particular does not comprise any substitution at any of positions 9, 12, 23, 26, 31, 87 and 95;
- more particularly does not comprise any substitution at at least one of the positions 9, 12, 23, 26, 31, 49, 52, 81, 84, 87, 95, 119, 123, 127, 131 and 132, and in particular does not comprise any substitution at any of positions 9, 12, 23, 26, 31, 49, 52, 81, 84, 87, 95, 119, 123, 127, 131 and 132;
the identified positions being determined by alignment with the sequence set forth as SEQ ID NO: 1.

An interleukin-2 variant according to the invention may comprise at least one, and in particular one, amino acid deletion at a position selected from S4, S5 or S6,
the indicated positions being determined by alignment with the sequence set forth as SEQ ID NO: 1.

An interleukin-2 variant according to the invention may in particular be selected from the group consisting of:
- a variant consisting in the amino acid substitution H16R and at least one, and in particular one, amino acid deletion at a position selected from S4, S5 or S6; or
- a variant consisting in the amino acid substitutions H16R and L19R and at least one, and in particular one, amino acid deletion at a position selected from S4, S5 or S6; or
- a variant consisting in the amino acid substitutions H16R and V91K and at least one, and in particular one, amino acid deletion at a position selected from S4, S5 or S6; or
- a variant consisting in the amino acid substitutions L19R and V91K and at least one, and in particular one, amino acid deletion at a position selected from S4, S5 or S6; or
- a variant consisting in the amino acid substitutions H16R, L19R and V91K and at least one, and in particular one, amino acid deletion at a position selected from S4, S5 or S6;
the identified positions being determined by alignment with the sequence set forth as SEQ ID NO: 1.

An interleukin-2 according to the invention may be a human IL-2 variant.

An interleukin-2 according to the invention may be an IL-2 agonist, the interleukin-2 variant being more particularly selected from the group consisting of:
- a variant comprising, and in particular consisting in, the amino acid substitution H16R;
- a variant comprising, and in particular consisting in, the amino acid substitutions H16R and L19R; and
- a variant comprising, and in particular consisting in, the amino acid substitutions L19R and V91K;
the identified positions being determined by alignment with the sequence set forth as SEQ ID NO: 1.

An interleukin-2 according to the invention may stimulate T regulatory cells.

An interleukin-2 according to the invention may be an IL-2 antagonist, the interleukin-2 variant being more particularly selected from the group consisting of:
- a variant comprising, and in particular consisting in, the amino acid substitutions H16R and V91K; and
- a variant comprising, and in particular consisting in, the amino acid substitutions H16R, L19R and V91K;
the identified positions being determined by alignment with the sequence set forth as SEQ ID NO: 1.

Another object of the present invention is an isolated polynucleotide encoding an interleukin-2 variant according to the invention.

The present invention also relates to a recombinant vector comprising at least one polynucleotide according to the invention.

The invention further relates to a host cell, in particular a non-human host cell, comprising at least one polynucleotide and/or at least one recombinant vector according to the invention.

Another object of the present invention is a pharmaceutical composition comprising, in a pharmaceutically acceptable carrier, an interleukin-2 variant according to the invention.

A further object of the present invention relates to the pharmaceutical composition according to the invention or an interleukin-2 variant according to the invention, in particular an antagonist IL-2 variant according to the invention, for use in the prevention or treatment of cancer in an individual in need thereof, in particular in a human being.

The cancer may in particular be selected from the group consisting of leukemias; seminomas; melanomas; teratomas; lymphomas, in particular non-Hodgkin lymphoma; neuroblastomas; gliomas; adenocarcinoma; mesothelioma, in particular pleural mesothelioma, peritoneal mesothelioma, pericardial mesothelioma and end stage mesothelioma; rectal cancer; endometrial cancer; thyroid cancer, in particular papillary thyroid carcinoma, follicular thyroid carcinoma, medullary thyroid carcinoma, undifferentiated thyroid cancer, multiple endocrine neoplasia type 2 A, multiple endocrine neoplasia type 2B, familial medullary thyroid cancer, pheochromocytoma and paraganglioma; skin cancer, in particular malignant melanoma, basal cell carcinoma, squamous cell carcinoma, Kaposi's sarcoma, keratoacanthoma, moles, dysplastic nevi, lipoma, angioma and dermatofibroma; nervous system cancer; brain cancer, in particular astrocytoma, medulloblastoma, glioma, lower grade glioma, ependymoma, germinoma, glioblastoma multiform, oligodendroglioma, schwannoma, retinoblastoma, congenital tumors, spinal cord neurofibroma, glioma or sarcoma; skull cancer, in particular osteoma, hemangioma, granuloma, xanthoma or osteitis deformans; meninges cancer, in particular meningioma, meningiosarcoma or gliomatosis; head and neck cancer, in particular head and neck squamous cell carcinoma and oral cancer, in particular buccal cavity cancer, lip cancer, tongue cancer, mouth cancer or pharynx cancer; lymph node cancer; gastrointestinal cancer; liver cancer, in particular hepatoma, hepatocellular carcinoma, cholangiocarcinoma, hepatoblastoma, angiosarcoma, hepatocellular adenoma and hemangioma; colon cancer; stomach or gastric cancer; esophageal cancer, in particular squamous cell carcinoma, larynx, adenocarcinoma, leiomyosarcoma or lymphoma; colorectal cancer; intestinal cancer; small bowel or small intestines cancer, in particular adenocarcinoma lymphoma, carcinoid tumors, Kaposi's sarcoma, leiomyoma, hemangioma, lipoma, neurofibroma or fibroma; large bowel or large intestines cancer, in particular adenocarcinoma, tubular adenoma, villous adenoma, hamartoma or leiomyoma; pancreatic cancer, in particular ductal adenocarcinoma, insulinoma, glucagonoma, gastrinoma, carcinoid tumors or vipoma; ear, nose and throat (ENT) cancer; breast cancer, in particular HER2-enriched breast cancer, luminal A breast cancer, luminal B breast cancer and triple negative breast cancer; cancer of the uterus, in particular endometrial cancer, more particularly endometrial carcinomas, endometrial stromal sarcomas and malignant mixed Miillerian tumors, uterine sarcomas, leiomyosarcomas and gestational trophoblastic disease; ovarian cancer, in particular dysgerminoma, Granulosa-Theca cell tumors and Sertoli-Leydig cell tumors; cervical cancer; vaginal cancer, in particular squamous-cell vaginal carcinoma, vaginal adenocarcinoma, clear cell vaginal adenocarcinoma, vaginal germ cell tumors, vaginal sarcoma botryoides and vaginal melanoma; vulvar cancer, in particular squamous cell vulvar carcinoma, verrucous vulvar carcinoma, vulvar melanoma, basal cell vulvar carcinoma, Bartholin gland carcinoma, vulvar adenocarcinoma and erythroplasia of Queyrat; genitourinary tract cancer; kidney cancer, in particular clear renal cell carcinoma, chromophobe renal cell carcinoma, papillary renal cell carcinoma, adenocarcinoma, Wilms tumor, nephroblastoma, lymphoma or leukemia; adrenal cancer; bladder cancer; urethra cancer, in particular squamous cell carcinoma, transitional cell carcinoma or adenocarcinoma; prostate cancer, in particular adenocarcinoma or sarcoma; testis cancer, in particular seminoma, teratoma, embryonal carcinoma, teratocarcinoma, choriocarcinoma, sarcoma, interstitial cell carcinoma, fibroma, fibroadenoma, adenomatoid tumors or lipoma; lung cancer, in particular small cell lung carcinoma (SCLC), non-small cell lung carcinoma (NSCLC), including squamous cell lung carcinoma, lung adenocarcinoma (LUAD), and large cell lung carcinoma, bronchogenic carcinoma, alveolar carcinoma, bronchiolar carcinoma, bronchial adenoma, lung sarcoma, chondromatous hamartoma and pleural mesothelioma; sarcomas, in particular Askin's tumor, sarcoma botryoides, chondrosarcoma, Ewing's sarcoma, malignant hemangioendothelioma, malignant schwannoma, osteosarcoma and soft tissue sarcomas; soft tissue sarcomas, in particular alveolar soft part sarcoma, angiosarcoma, cystosarcoma phyllodes, dermatofibrosarcoma protuberans, desmoid tumor, desmoplastic small round cell tumor, epithelioid sarcoma, extraskeletal chondrosarcoma, extraskeletal osteosarcoma, fibrosarcoma, gastrointestinal stromal tumor (GIST), hemangiopericytoma, hemangiosarcoma, Kaposi's sarcoma, leiomyosarcoma, liposarcoma, lymphangio sarcoma, lymphosarcoma, malignant peripheral nerve sheath tumor (MPNST), neurofibrosarcoma, plexiform fibrohistiocytic tumor, rhabdomyosarcoma, synovial sarcoma and undifferentiated pleomorphic sarcoma; cardiac cancer, in particular angiosarcoma, fibrosarcoma, rhabdomyosarcoma, liposarcoma, myxoma, rhabdomyoma, fibroma, lipoma and teratoma; bone cancer, in particular osteogenic sarcoma, osteosarcoma, fibrosarcoma, malignant fibrous histiocytoma, chondrosarcoma, Ewing's sarcoma, malignant lymphoma and reticulum cell sarcoma, multiple myeloma, malignant giant cell tumor chordoma, osteochronfroma, osteocartilaginous exostoses, benign chondroma, chondroblastoma, chondromyxoid fibroma, osteoid osteoma and giant cell tumors; hematologic and lymphoid cancer; blood cancer, in particular acute myeloid leukemia, chronic myeloid leukemia, acute lymphoblastic leukemia, chronic lymphocytic leukemia, myeloproliferative diseases, multiple myeloma and myelodysplasia syndrome; Hodgkin's disease; non-Hodgkin's, and the metastases thereof.

A pharmaceutical composition according to the invention or an interleukin-2 variant according to the invention, in particular an antagonist IL-2 variant according to the invention, may be administered to the individual in need thereof in combination with at least one immune check-point inhibitor; preferably anti-PD-1 and/or anti-PDL-1.

A further object of the present invention relates to the pharmaceutical composition according to the invention or an interleukin-2 variant according to the invention, in particular an agonist IL-2 variant according to the invention, for use in the prevention or treatment of a disease selected from the group consisting of acute or chronic inflammatory diseases, allergic diseases, autoimmune diseases, graft-versus-host disease and graft rejection.

### BRIEF DESCRIPTION OF THE FIGURES

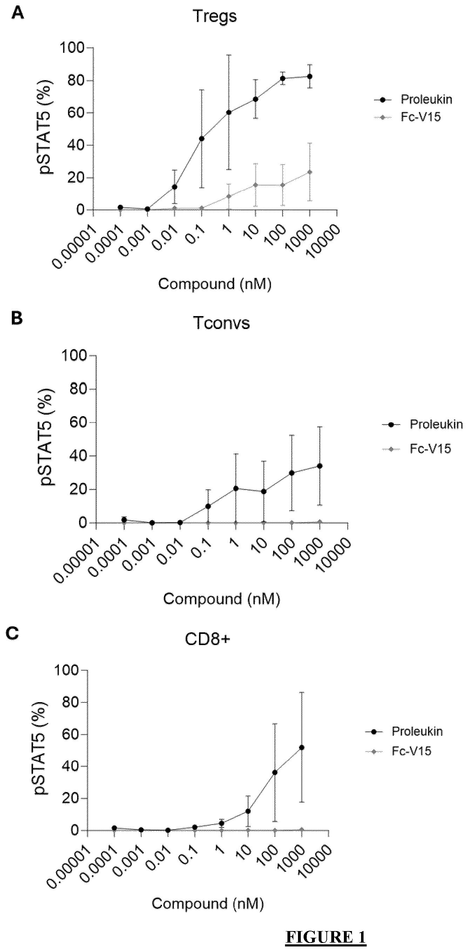

**Figure 1** shows phosphorylation of STATS (pSTAT5 (%) - ordinate) in human Tregs (IL-2Rabg) (Figure 1A), CD4+ Tconvs (IL-2Rbg) (Figure 1B) and CD8+ T cells (IL-2Rbg) (Figure 1C) from human PBMCs, measured by flow cytometry after incubation with different concentrations of Proleukine or Fc-V15 (from 0,0001 nM to 1000 nM, expressed as Proleukin equivalent concentration, corresponding to 0,00005 nM to 500 nM of FcV15 - abscissa). Average ± SEM from two independent donors.

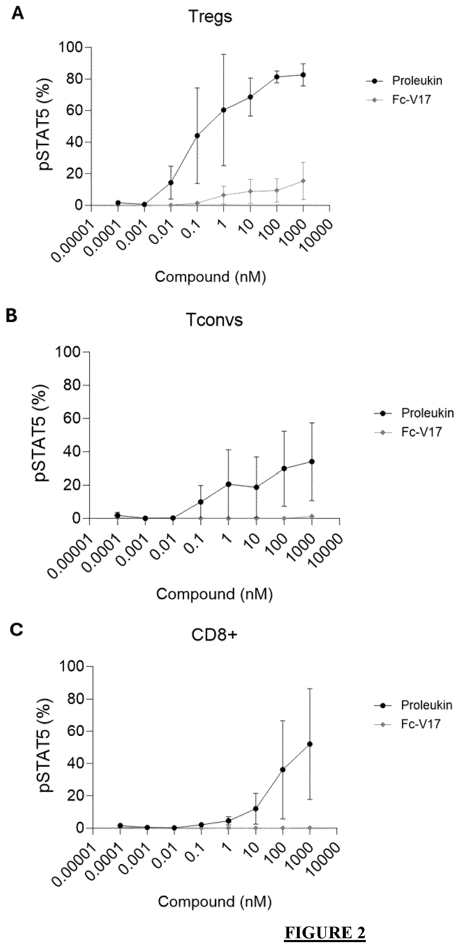

**Figure 2** shows phosphorylation of STATS (pSTAT5 (%) - ordinate) in human Tregs (IL-2Rabg) (Figure 2A), CD4+ Tconvs (IL-2Rbg) (Figure 2B) and CD8+ T cells (IL-2Rbg) (Figure 2C) from human PBMCs, measured by flow cytometry after incubation with different concentrations of Proleukine or Fc-V17 (from 0,0001 nM to 1000 nM, expressed as Proleukin equivalent concentration, corresponding to 0,00005 nM to 500 nM of FcV17 - abscissa). Average ± SEM from two independent donors.

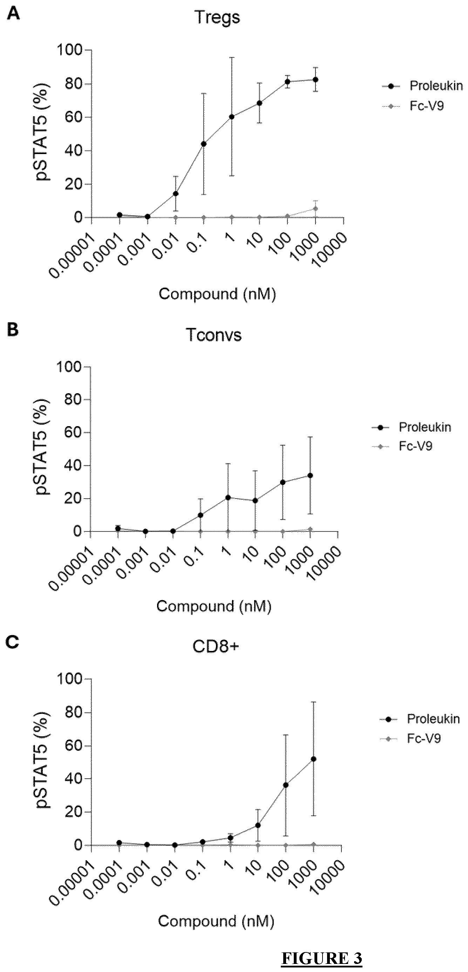

**Figure 3** shows phosphorylation of STATS (pSTAT5 (%) - ordinate) in human Tregs (IL-2Rabg) (Figure 3A), CD4+ Tconvs (IL-2Rbg) (Figure 3B) and CD8+ T cells (IL-2Rbg) (Figure 3C) from human PBMCs, measured by flow cytometry after incubation with different concentrations of Proleukine or Fc-V9 (from 0,0001 nM to 1000 nM, expressed as Proleukin equivalent concentration, corresponding to 0,00005 nM to 500 nM of FcV9 - abscissa). Average ± SEM from two independent donors.

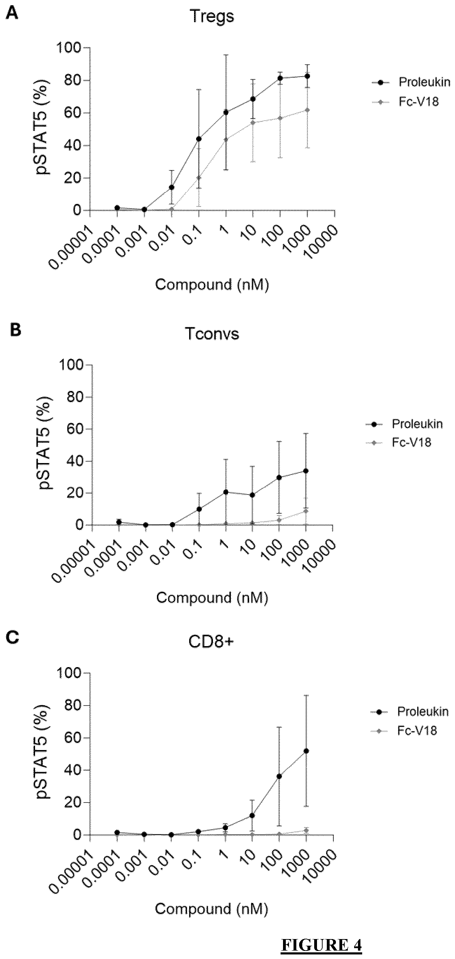

**Figure 4** shows phosphorylation of STATS (pSTAT5 (%) - ordinate) in human Ts (IL-2Rabg) (Figure 4A), CD4+ Tconvs (IL-2Rbg) (Figure 4B) and CD8+ T cells (IL-2Rbg) (Figure 4C) from human PBMCs, measured by flow cytometry after incubation with different concentrations of Proleukine or Fc-V18 (from 0,0001 nM to 1000 nM, expressed as Proleukin equivalent concentration, corresponding to 0,00005 nM to 500 nM of FcV18 - abscissa). Average ± SEM from two independent donors.

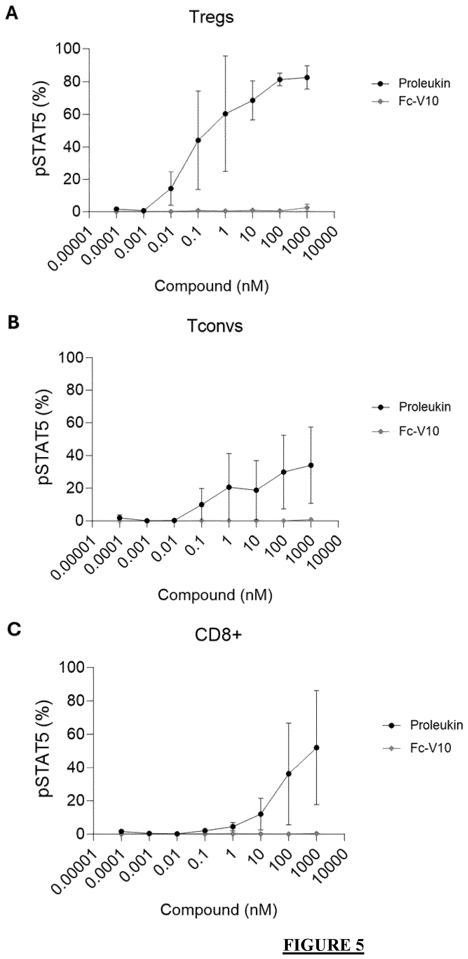

**Figure 5** shows phosphorylation of STATS (pSTAT5 (%) - ordinate) in human Ts (IL-2Rabg) (Figure 5A), CD4+ Tconvs (IL-2Rbg) (Figure 5B) and CD8+ T cells (IL-2Rbg) (Figure 5C) from human PBMCs, measured by flow cytometry after incubation with different concentrations of Proleukine or Fc-V10 (from 0,0001 nM to 1000 nM, expressed as Proleukin equivalent concentration, corresponding to 0,00005 nM to 500 nM of FcV10 - abscissa). Average ± SEM from two independent donors.

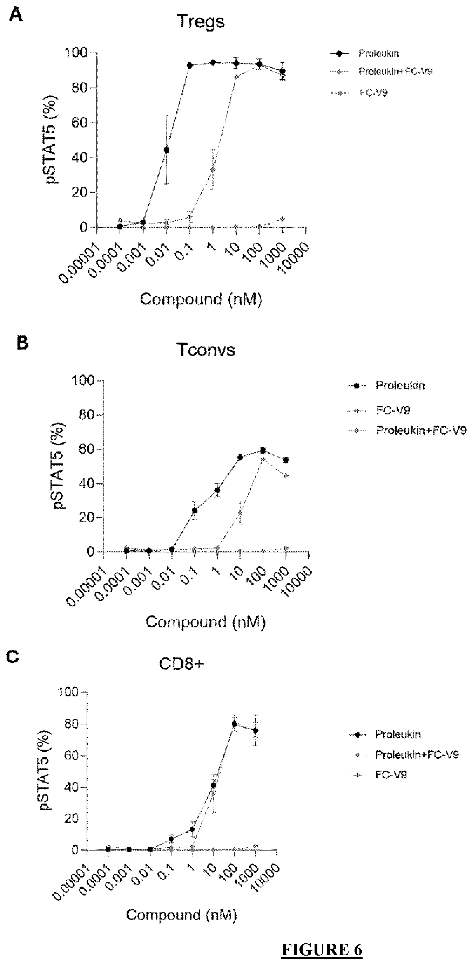

**Figure 6** shows phosphorylation of STATS (pSTAT5(%) - ordinate) in human Tregs (IL-2Rabg) (Figure 6A), CD4+ Tconv (IL-2Rbg) (Figure 6B) and CD8+ T cells (IL-2Rbg) (Figure 6C) from human PBMCs, measured by flow cytometry after incubation with different concentrations of Proleukine alone, Proleukine in the presence of a fixed concentration of Fc-V9 (500nM), or Fc-V9 alone (from 0,0001 nM to 1000 nM - abscissa). Average ± SEM from two independent donors.

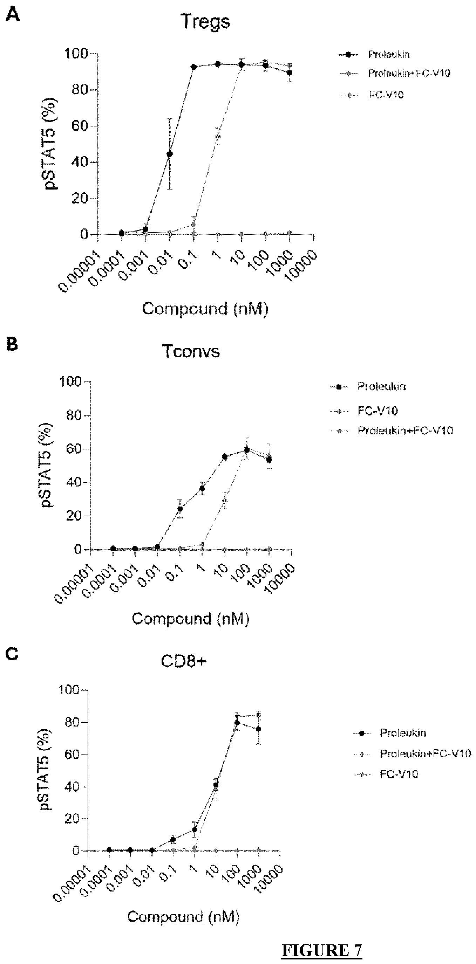

**Figure 7** shows phosphorylation of STATS (pSTAT5(%) - ordinate) in human Tregs (IL-2Rabg) (Figure 7A), CD4+ Tconv (IL-2Rbg) (Figure 7B) and CD8+ T cells (IL-2Rbg) (Figure 7C) from human PBMCs measured by flow cytometry after incubation with different concentrations of Proleukine alone, Proleukine in the presence of a fixed concentration of Fc-V10 (500nM), orFc-V10 alone (from 0,0001 nM to 1000 nM - abscissa). Average ± SEM from two independent donors.

### DETAILED DESCRIPTION OF THE INVENTION

### IL-2 variants according to the invention

The invention relates to interleukin-2 (IL-2) variants capable of preferentially stimulating T-regulatory cells (IL-2 agonists) or antagonizing IL-2 (IL-2 antagonists).

The term interleukin-2 or IL-2, also known as TCGF or lymphokine, refers to a protein encoded by the IL-2 gene in a mammalian genome. IL-2 is expressed as a precursor containing a N-terminal signal peptide (20 amino acids) which is cleaved to yield the mature protein (IL-2). Representative examples of IL-2 include, without limitation, human (Gene ID: 3558), rat (Gene ID: 116562), cat (Gene ID: 751114), and mouse (Gene ID 16183) forms. As used herein, IL-2 refers to wild-type IL-2 and in particular to wild-type human IL-2. Human IL-2 precursor has the 153 amino acid sequence UniProtKB/Swiss-Prot: P60568.1. Human mature IL-2 has the 133 amino acid sequence from positions 21 to 153 of the precursor and corresponds to SEQ ID NO: 1 referred to herein.

In the following description, the residues are designated by the standard one letter amino acid code and the indicated positions are determined by alignment with SEQ ID NO: 1. For example, K9 is the lysine residue at position 9 of SEQ ID NO: 1. Substitutions are designated herein by the one letter amino acid code followed by the substituting residue in one letter amino acid code; K9E is a substitution of the lysine (K) residue at position 9 of SEQ ID NO: 1 with a Glutamic acid (E) residue.
"a", "an", and "the" include plural referents, unless the context clearly indicates otherwise. As such, the term "a" (or "an"), "one or more" or "at least one" can be used interchangeably herein; unless specified otherwise, "or" means "and/or".

The invention provides interleukin-2 (IL-2) variants, preferably human IL-2 variants, capable of preferentially stimulating T-regulatory cells (Tregs) (IL-2 agonists) or antagonizing IL-2 (IL-2 antagonists) that are useful for the treatment of immune disorders.

As used herein, *"immune disorders"* refer to diseases involving an immune dysfunction or immune dysregulation.

Immune dysfunction may comprise inhibition, dysfunction or overactivity of the immune system. Immune disorders include diseases that can be prevented or treated by immunomodulation using immunomodulatory, immunosuppressive, and/or immunostimulatory agents. Immunomodulation may comprise preferentially stimulating Tregs or inhibiting Tregs.

Immunosuppression may comprise preferentially stimulating Tregs, which in turn inhibit the immune effector cells or inhibiting immune effector cells directly.

Likewise, immunostimulation may comprise inhibiting Tregs, which in turn unleash immune cells from immunosuppression or stimulating effector immune cells directly. Immunomodulatory agents which inhibit Tregs and immunostimulatory agents are useful for preventing or treating diseases where inhibition of Tregs and/or stimulation of immune responses is beneficial such, as with no limitations, infectious diseases and cancer, including the treatment of infectious diseases and cancer and the increase of immune response against vaccines. Vaccines are in particular vaccines directed to cancer or infectious diseases.

Immunomodulatory agents which stimulate Tregs and immunosuppressive agents are useful for treating diseases involving an immune dysfunction such as, with no limitations, allergic and autoimmune diseases and diseases associated with overactivity of the immune system, such as for example chronic or acute inflammatory diseases, graft-versus-host disease (GVHD) including acute GVHD and graft rejection.

The IL-2 variants according to the invention which preferentially stimulate T-regulatory cells (Treg-agonist variants) are useful for expanding Tregs *ex vivo* or *in vivo.* The IL-2 variants according to the invention which antagonize IL-2 (IL-2-antagonist variants) are useful for blocking IL-2 -mediated overactivation of the immune system, or for inhibiting Tregs by depriving them from IL-2 signaling and consequently, stimulating immune responses including B, NK, CD4+ or CD8+ T cells, DC, macrophages and others, in particular CD8+ T cell immune response *in vivo,* such as anti-tumoral immune responses or immune responses against vaccines or pathogens. As used herein, *"preferentially stimulate T-regulatory cells"* means that the IL-2 variant promotes the proliferation or activation of T-regulatory cells over non-regulatory T cells.

As used herein *"T-regulatory cells", "Treg"* or *"Tregs"* refer to CD3+CD4+Foxp3+ T cells, including CD3+CD4+Foxp3+CD25+ and CD3+CD4+Foxp3+CD25- cells.

As used herein, *"T-effector cells", "Teff"* or *"Teffs"* refer to one or more of Tconv cells (CD3+CD4+Foxp3-); CD8+ T cells (CD3+CD8+) and NK cells (CD3-CD16+).

As used herein *"inhibit Tregs"* means that the IL-2 variant is capable of inhibiting the proliferation, the activation, or the suppressive function of Tregs cells by depriving them from IL-2 signaling. The inhibition of Tregs include the inhibition of Treg function or loss of Treg function, in particular Treg immunosuppressive function and the elimination of Tregs. The ability of the IL-2 variant of the invention to preferentially stimulate T-regulatory cells can be measured by standard assays that are well-known in the art and disclosed in the examples of the present application, including with no limitation STATS phosphorylation assay or flow cytometry analysis on a population of T cells from a subject treated *in vivo* or a peripheral blood sample treated *in vitro.* The ability of IL-2 variant to antagonize IL-2 can be determined by standard assays that are well-known in the art and disclosed in the examples of the present application, such as STATS phosphorylation assay in competition with Proleukine on a peripheral blood sample treated *in vitro* or quantification of proliferation using a CellTrace Violet (CTV) dilution assay.

The Treg-agonist variants are useful for reducing immune activation indirectly, by preferentially activating Tregs that in turn inhibit the immune effector cells. The Treg-agonist variants of the invention are thus useful for treating diseases where immunomodulation or immunosuppression is beneficial such as with no limitations, allergic and autoimmune diseases, and diseases comprising overactivity of the immune system including with no limitations chronic or acute inflammatory diseases, graft-versus-host disease (GVHD) and graft rejection.

The IL-2 variants according to the invention which are IL-2 antagonists are useful for inhibiting Tregs and consequently stimulating immune responses (B, NK, CD4+ or CD8+ T cells, DC, macrophages and others). The IL-2-antagonist variants according to the invention are useful for stimulating anti-tumoral immune response(s) or immune response(s) against a pathogen or a vaccine including a vaccine against cancer or infectious disease, in particular anti-tumoral CD8+ T-cell response and anti-CD8+ T-cell response against a pathogen or vaccine.

As mentioned above, an interleukin-2 (IL-2) variant according to the invention comprises:
(i) the amino acid substitution H16R; or
(ii) the amino acid substitution H16R and one or two amino acid substitution(s) selected from the group consisting of L19R and V91K; or
(iii) the amino acid substitution L19R and the amino acid substitution V91K;

the identified positions being determined by alignment with the sequence set forth as SEQ ID NO: 1,
the IL-2 variant comprising at most 10 substitutions compared with sequence SEQ ID NO: 1.

In particular, an IL-2 variant according to the invention comprises from 1 to 8 substitutions compared with SEQ ID NO: 1, more particularly from 1 to 5 substitutions and even more particularly from 1 to 3 substitutions compared with SEQ ID NO: 1.

An IL-2 variant according to the invention may comprise at least one, and in particular one, amino acid deletion at a position selected from S4, S5 or S6, the indicated positions being determined by alignment with wild-type human IL-2 set forth as sequence SEQ ID NO: 1.

In particular, an IL-2 variant according to the invention comprises from 1 to 8 substitutions compared with SEQ ID NO: 1, more particularly from 1 to 5 substitutions and even more particularly from 1 to 3 substitutions compared with SEQ ID NO: 1.

Preferably, an IL-2 variant according to the invention comprises:
- from 1 to 8 substitutions compared with SEQ ID NO: 1, more particularly from 1 to 5 substitutions and even more particularly from 1 to 3 substitutions compared with SEQ ID NO: 1; and
- at least one, and in particular one, amino acid deletion at a position selected from S4, S5 or S6, the indicated positions being determined by alignment with SEQ ID NO: 1.

In particular, an IL-2 variant according to the invention comprises:
- the amino acid substitution H16R; or
- the amino acid substitutions H16R and L19R; or
- the amino acid substitutions H16R and V91K; or
- the amino acid substitutions L19R and V91K; or
- the amino acid substitutions H16R, L19R and V91K;

the identified positions being determined by alignment with the sequence set forth as SEQ ID NO: 1,
the IL-2 variant comprising at most 10 substitutions compared with sequence SEQ ID NO: 1, in particular from 1 to 8 substitutions compared with SEQ ID NO: 1, more particularly from 1 to 5 substitutions and even more particularly from 1 to 3 substitutions compared with SEQ ID NO: 1.

Interleukin-2 variants according to the invention preferably do not comprise any substitution at at least one of the positions selected from the group consisting of positions 9, 12, 23, 26, 31, 87 and 95, and in particular does not comprise any substitution at any of positions 9, 12, 23, 26, 31, 87 and 95. More particularly, Interleukin-2 variants according to the invention preferably do not comprise any substitution at at least one of the positions 9, 12, 23, 26, 31, 49, 52, 81, 84, 87, 95, 119, 123, 127, 131 and 132, and in particular does not comprise any substitution at any of positions 9, 12, 23, 26, 31, 49, 52, 81, 84, 87, 95, 119, 123, 127, 131 and 132.

More particularly, an IL-2 variant according to the invention comprises as sole substitution(s) compared with SEQ ID NO: 1:
- the amino acid substitution H16R; or
- the amino acid substitutions H16R and L19R; or
- the amino acid substitutions H16R and V91K; or
- the amino acid substitutions L19R and V91K; or
- the amino acid substitutions H16R, L19R and V91K;
the identified positions being determined by alignment with the sequence set forth as SEQ ID NO: 1.

More particularly, an IL-2 variant according to the invention is selected from the group consisting of:
- a variant consisting in the amino acid substitution H16R and at least one, and in particular one, amino acid deletion at a position selected from S4, S5 or S6; or
- a variant consisting in the amino acid substitutions H16R and L19R and at least one, and in particular one, amino acid deletion at a position selected from S4, S5 or S6; or
- a variant consisting in the amino acid substitutions H16R and V91K and at least one, and in particular one, amino acid deletion at a position selected from S4, S5 or S6; or
- a variant consisting in the amino acid substitutions L19R and V91K and at least one, and in particular one, amino acid deletion at a position selected from S4, S5 or S6; or
- a variant consisting in the amino acid substitutions H16R, L19R and V91K and at least one, and in particular one, amino acid deletion at a position selected from S4, S5 or S6;
the identified positions being determined by alignment with the sequence set forth as SEQ ID NO: 1.

An IL-2 variant according to the invention may in particular be a human IL-2 variant.

As previously indicated, the invention provides IL-2 variants, preferably human IL-2 variants, capable of preferentially stimulating T-regulatory cells (Tregs) (IL-2 agonists) or antagonizing IL-2 (IL-2 antagonists) that are useful for the treatment of immune disorders.

Accordingly, an IL-2 variant according to the invention can be an IL-2 agonist. An IL-2 agonist according to the invention may more particularly be selected from the group consisting of:
- a variant comprising the amino acid substitution H16R, and in particular a variant consisting in the amino acid substitution H16R and at least one, and in particular one, amino acid deletion at a position selected from S4, S5 or S6;
- a variant comprising the amino acid substitutions H16R and L19R, and in particular a variant consisting in the amino acid substitutions H16R and L19R and at least one, and in particular one, amino acid deletion at a position selected from S4, S5 or S6; and
- a variant comprising the amino acid substitutions L19R and V91K, and in particular a variant consisting in the amino acid substitutions L19R and V91K and at least one, and in particular one, amino acid deletion at a position selected from S4, S5 or S6;
the identified positions being determined by alignment with the sequence set forth as SEQ ID NO: 1.

An IL-2 variant according to the invention is in particular an IL-2 variant which stimulates T regulatory cells.

Alternatively, an interleukin-2 variant according to the invention may be an IL-2 antagonist.

An IL-2 antagonist according to the invention may in particular be selected from the group consisting of:
- a variant comprising the amino acid substitutions H16R and V91K, and in particular a variant consisting in the amino acid substitutions H16R and V91K and at least one, and in particular one, amino acid deletion at a position selected from S4, S5 or S6; and
- a variant comprising the amino acid substitutions H16R, L19R and V91K, and in particular a variant consisting in the amino acid substitutions H16R, L19R and V91K and at least one, and in particular one, amino acid deletion at a position selected from S4, S5 or S6;
the identified positions being determined by alignment with the sequence set forth as SEQ ID NO: 1.

In the various embodiments, the IL-2 variant (IL-2 agonist variant or IL-2 antagonist variant) may comprise at least one additional amino acid mutation (insertion, deletion, substitution) or not.

In preferred embodiments, an IL-2 variant of the invention as defined above does not comprise additional amino acid mutations.

In some other embodiments, the IL-2 variant comprises at least one, and in particular one, two or three, more particularly one or two, even more particularly one, additional amino acid mutation (insertion, deletion, substitution).

An IL-2 variant of the invention is at least 125 amino acids in size. Preferably, an IL-2 variant of the invention is at least 130 or more amino acids in size. In particular, an IL-2 variant of the invention is 132 amino acids in size.

In some preferred embodiments, an IL-2 variant of the invention has at least 90% amino acid identity with SEQ ID NO: 1. Preferably, said IL-2 variant of the invention has at least 91%, 92%, 93%, 94%, 95%, 96%, 97%, 98% or 99% sequence identity with said sequence, even more preferably at least 95%, 96%, 97%, 98% or 99% 98% or 99% identity with said sequence.

The terms *"sequence homology"* or *"sequence identity"* or *"homology"* or *"identity"* are used interchangeably herein. For the purpose of the invention, it is defined here that in order to determine the percentage of sequence homology or sequence identity of two amino acid sequences or of two nucleic acid sequences, the sequences are aligned for optimal comparison purposes. In order to optimize the alignment between the two sequences gaps may be introduced in any of the two sequences that are compared. Such alignment can be carried out over the full length of the sequences being compared. Alternatively, the alignment may be carried out over a shorter length, for example over about 20, about 50, about 100 or more nucleic acids/based or amino acids. The sequence identity is the percentage of identical matches between the two sequences over the reported aligned region. A comparison of sequences and determination of percentage of sequence identity between two sequences can be accomplished using a mathematical algorithm. The skilled person will be aware of the fact that several different computer programs are available to align two sequences and determine the identity between two sequences (Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley).

The percent sequence identity between two amino acid sequences or between two nucleotide sequences may be determined using the Needleman and Wunsch algorithm for the alignment of two sequences. (Needleman and Wunsch, J. Mol. Biol., 48: 443-453 (1970)). Both amino acid sequences and nucleotide sequences can be aligned by the algorithm. The Needleman-Wunsch algorithm has been implemented in the computer program NEEDLE.

For the purpose of the invention, the NEEDLE program from the EMBOSS package was used (version 2.8.0 or higher, EMBOSS: The European Molecular Biology Open Software Suite (2000) Rice, P. Longden J. and Bleasby, A. Trends in Genetics 16, (6) pp276- 277, http://emboss.bioinformatics.nl/). For protein sequences EBLOSUM62 is used for the substitution matrix. For nucleotide sequence, EDNAFULL is used. The optional parameters used are a gap opening penalty of 10 and a gap extension penalty of 0.5. No end gap penalty is added. In the Output section, Yes has been indicated in response to the question "Brief identity and similarity" and "SRS pairwise" indicated as Output alignment format.

After alignment by the program NEEDLE as described above the percentage of sequence identity between a query sequence and a sequence of the invention is calculated as follows: Number of corresponding positions in the alignment showing an identical amino acid or identical nucleotide in both sequences divided by the total length of the alignment after subtraction of the total number of gaps in the alignment. The identity defined as herein can be obtained from NEEDLE by using the NOBRIEF option and is labeled in the output of the program as "longest-identity".

The similarity of nucleotide and amino acid sequences, i.e. the percentage of sequence identity, can be determined via sequence alignments using several other art-known algorithms, preferably with the mathematical algorithm of Karlin and Altschul (Karlin and Altschul, Proc. Natl. Acad. Sci. USA, 90: 5873-5877 (1993)), with hmmalign (HMMER package, http://hmmer.org/) or with the CLUSTAL algorithm (Thompson et al., Nucleic Acids Res., 22: 4673-80 (1994)) available e.g. on https://www.ebi.ac.uk/Tools/msa/clustalo/ or the GAP program (mathematical algorithm of the University of Iowa) or the mathematical algorithm of Myers and Miller (1989 - Cabios 4: 11-17) or Clone Manager 9. Preferred parameters used are the default parameters as they are set on https://www.ebi.ac.uk/Tool s/msa/clustalo/.

The grade of sequence identity (sequence matching) may be calculated using e.g. BLAST, BLAT or BlastZ (or BlastX). A similar algorithm is incorporated into the BLASTN and BLASTP programs of Altschul et al., J. Mol. Biol., 215: 403-410 (1990). BLAST polynucleotide searches are performed with the BLASTN program, score = 100, word length = 12, to obtain polynucleotide sequences that are homologous to those nucleic acids which encode the relevant protein.

BLAST protein searches are performed with the BLASTP program, score = 50, word length = 3, to obtain amino acid sequences homologous to the SHC polypeptide. To obtain gapped alignments for comparative purposes, Gapped BLAST is utilized as described in Altschul et al., Nucleic Acids Res., 25: 3389-3402 (1997). When utilizing BLAST and Gapped BLAST programs, the default parameters of the respective programs are used. Sequence matching analysis may be supplemented by established homology mapping techniques like Shuffle-LAGAN (Brudno, Bioinformatics, 19 Suppl 1: 154-162 (2003)) or Markov random fields. When percentages of sequence identity are referred to in the present application, these percentages are calculated in relation to the full length of the longer sequence, if not specifically indicated otherwise.

In particular embodiments, % identity between two sequences is determined using CLUSTAL O (version 1.2.4).

The invention encompasses IL-2 variants of the invention having one or more modifications into one or more amino acid residues, peptide bonds, N-and/or C-terminal ends, as long as the modified variant is functional (i.e., capable of preferentially stimulating Tregs or antagonizing IL-2). Preferred modifications are those which increase the stability or the bioavailability of the IL-2 variant according to the invention, in particular which increase its half-life in vivo; decrease its immunogenicity; or facilitate its purification, detection or targeting to a specific cell type or tissue. These modifications which are introduced into the variant by the conventional methods known to those skilled in the art, include, in a non-limiting manner: mutation (insertion, deletion, substitution) of one or more amino acids in the amino acid sequence; fusion to an amino acid moiety of interest (protein of interest for a therapeutic use or tag for the purification, the detection (antibody epitope or label) or coupling to a molecule or agent of interest; substitution of a natural amino acid with a non-proteinogenic amino acid (D amino acid or amino acid analog); modification of the peptide bond, in particular with a bond of the retro or retro-inverso type or a bond different from the peptide bond; the cyclization, and the addition of a chemical group to the amino acid side chain or the N-and/or C-terminal end(s) of the variant, in particular for coupling to a molecule or agent of interest to the variant. The modifications include in a non-limiting manner, esterification, glycosylation, acylation such as acetylation or linking myristic acid, amidation, phosphorylation, biotinylation, PEGylation, coupling of farnesyl and similar modifications which are well-known in the art. Modifications can be introduced at the N-terminus (acetylation), the C-terminus (amidation) of the IL-2 variant or if deemed suitable, also to any amino acid other than the terminal amino acids (e.g. farnesyl coupling to a cysteine side chain). Conversion of the acid function on the C-terminus into an aldehyde and alkylation of the thiol function of a cysteine residue are used for chemo selective ligation or the formation of reduced peptide bonds.

In particular, the invention encompasses IL-2 variants comprising or consisting of a chain of natural amino acids (20 gene-encoded amino acids (A, R, N, D, C, Q, E, G, H, I, L, K, M, F, P, S, T, W, X and Y) in a L- and/or D-configuration) linked via a peptide bond and furthermore comprises peptidomimetics of such variants where the amino acid(s), peptide bond(s) N-and/or C-terminal ends have been replaced by functional analogues. Such functional analogues of amino acids include all known amino acids other than said 20 gene-encoded amino acids.

In some embodiments the IL-2 variant is provided in a form where it is associated with at least an agent of interest, for example in the form of a complex such as a molecular complex or a particle; a conjugate or a fusion protein. The agent of interest includes with no limitation any therapeutic agent including a cell such as patient's Chimeric Antigen Receptor (CAR) T-cell, any agent that increases the stability or the bioavailability of the IL-2 variant according to the invention, in particular that increases its half-life in vivo; any agent that decreases its immunogenicity; or any agent that facilitates its purification, detection or its targeting to a specific cell type or tissue. The agent of interest can be a small or large chemical compound, a macromolecule or a particle. Particles include with no limitation liposomes, micelles and nanoparticles including liposomes, micelles and nanoparticles carrying an active agent such as nanocarriers. For example, the IL-2 variant, and eventually other agent(s) of interest can be encapsulated into the particles or grafted onto said particles by means well-known in the art. In a preferred embodiment, the agent of interest is selected from the group consisting of peptides; proteins including antibodies; bioactive substances like drugs for the treatment of human, or animal diseases; labels, tags and particles.

In some more embodiments, the agent of interest is an anti-IL-2 antibody, preferably an anti-IL-2 antibody with pro-Treg or pro-T-effector function or a functional fragment thereof comprising at least the antigen binding site; more preferably a human or humanized anti-IL-2 antibody. Anti-IL-2 antibodies with pro-Treg function or fragment thereof are useful for the treatment of diseases associated with overactivity of the immune system, such as with no limitations, chronic or acute inflammatory diseases, graft-versus-host disease (GVHD) and graft rejection. Anti-IL-2 antibodies with pro-T-effector function or fragment thereof are useful for preventing or treating immune disorders where Treg inhibition and/or stimulation of a CD8+ T-cell response is beneficial such as with no limitations cancer, infectious diseases and vaccination. In some embodiments, the anti-IL-2 antibody, preferably an anti-IL-2 antibody with pro-Treg or pro-T-effector function, is a whole antibody molecule.

In some more preferred embodiments, the agent of interest comprises or consists of a ligand of a surface molecule specific for Tregs. Surface molecules specific for Tregs are known in the art and disclosed for example in Bhairavabhotla et al., Human Immunol., 2016, 77, 201-13; van der Veeken et al., Cold Spring Harb. Symp. Quant Biol., 2013, 78, 215-22; Pfoertner et al., Genome Biol., 2006, 7, R54; Sugimoto et al., Int. Immunol., 2006, 18, 1197-. Non-limiting examples of surface molecules specific for Tregs that can be used in the invention include CD25, CTLA-4, CCR8, ICOS, CD70, GARP, IL1R1, CD39, CCR4, and CD177.

The agent of interest may in particular be an antibody against said surface molecule specific for Tregs or a functional fragment thereof comprising at least the antigen binding site; preferably an anti-CTLA-4, anti-CD25, anti-CCR8, anti-ICOS, anti-CD70, anti-GARP, anti-ILlRl, anti-CD39, anti-CCR4 or anti-CD177 antibody; more preferably a human or humanized antibody. In some embodiments, the antibody against the Treg specific surface molecule is a whole antibody molecule, preferably whole human or humanized antibody molecule.

In some more preferred embodiments, the agent of interest is selected from the group comprising: whole antibody or antibody Fc region, preferably whole human antibody or human Fc region; multi- specific antigen binding protein such as bispecific antibody; Ankyrin and Designed Ankyrin Repeat Protein (DARPin); MHC-peptide multimer such as class I or class II MHC-peptide tetramer, in particular MHC-peptide multimer. The peptide in the MHC-peptide multimer may be derived from an antigen capable of inducing auto-immune or allo-immune response(s) such as a self-antigen or from a foreign antigen such as an antigen from a pathogen or a tumor, in particular a vaccine antigen against a pathogen or a tumor.

It is within the present invention that the complex, conjugate or fusion protein comprises more than one IL-2 variant according to the present invention, i.e., a plurality of such variants, whereby the plurality of the variants may comprise a plurality of the same or of different variants. Also, the complex, conjugate or fusion protein according to the present invention may also comprise more than one agent of interest, whereby the plurality of the agents may comprise a plurality of the same or of different agents.

The agent of interest is covalently or non-covalently linked to the IL-2 variant of the invention. The agent of interest may be coupled to the IL-2 variant, directly or indirectly. Indirect coupling of the agent of interest to the IL-2 variant may be through a linker that is attached to the IL-2 variant of the invention. Linkers, also named spacers, that can be used to physically separate the IL-2 variant of the invention to the agent of interest agent are known in the art and include a peptide bond, an amino acid, a peptide of appropriate length or a different molecule providing the desired feature. The linker may be attached to the N-terminus, the C-terminus of the IL-2 variant or if deemed suitable, also to any amino acid other than the terminal amino acids. The IL-2 variant of the invention can be chemically linked to the agent of interest by covalent bonds using standard conjugation techniques. The agent of interest can be linked to the N-terminus, the C-terminus of the IL-2 variant, or if applicable, to any amino acid other than the terminal amino acids. Functional groups, modifications also called derivatizations or a linker may also be introduced into the IL-2 variant for conjugating the IL-2 variant to the agent of interest. Such covalent bonds are preferably formed between, either a suitable reactive group of the IL-2 variant and the agent of interest, and more preferably between a terminus of the IL-2 variant according to the present invention and the agent of interest. Depending on the chemical nature of the agent of interest, the moiety, group or radical with which such covalent bond is formed varies and it is within the skills of a person of the art to create such bond. Chemical linkage may be via a disulphide bond, thioether, thiol-maleimide or amide linkage. Other ways of linking the IL-2 variant to the cargo include use of a C-terminal aldehyde to form an oxime, use of a click reaction or formation of a morpholino linkage with a basic amino acid on the peptide.

When the agent of interest is a peptide or a protein including an antibody or functional fragment thereof, the IL-2 variant is advantageously provided as a chimeric fusion protein comprising the heterologous agent of interest (different from IL-2 or IL-2 fragment) fused to the N-terminus or the C-terminus or inserted into the amino acid sequence of the IL-2 variant of the invention, directly or via a peptide spacer as described above. The fusion protein is expressed from a chimeric construct in which a nucleotide sequence encoding the IL-2 variant of the invention is fused in frame to a nucleotide sequence encoding the peptide/protein of interest, using standard recombinant DNA techniques. The resulting fusion protein/peptide is of heterologous origin, i.e., it is different from naturally occurring peptides or proteins such as IL-2 or other cytokines of the same family.

The IL-2 variant can also be linked to the agent of interest (molecule or particle carrying the molecule) via non-covalent bounds such as ionic bonds, hydrogen bonds or hydrophobic interactions or a combination of such bonds. Non-limitative examples include antigen- antibody interaction between the IL-2 variant and an anti-IL-2 antibody; streptavidin-biotin interactions between a biotinylated IL-2 variant and an agent of interest (for example nanoparticles like Quantum dots) that is conjugated to streptavidin or a biotinylated agent and an IL-2 variant that is conjugated to streptavidin.

In preferred embodiments, the IL-2 variant is provided as a chimeric fusion protein comprising a heterologous protein or peptide of interest fused to the N-terminus or the C-terminus or inserted into the amino acid sequence of the IL-2 variant of the invention, directly or via a peptide spacer. The protein or peptide of interest is preferably selected from the group comprising: whole antibody or antibody Fc region, preferably whole human antibody or human Fc region; multi- specific antigen binding protein such as bispecific antibody; Ankyrin and Designed Ankyrin Repeat Protein (DARPin); MHC-peptide multimer such as class I or class II MHC-peptide tetramer, in particular MHC-peptide multimer wherein the peptide is derived from an antigen capable of inducing auto-immune or allo-immune response(s) such as a self-antigen. In some more preferred embodiments, the antibody is an antibody against a surface molecule specific for Tregs or a functional fragment thereof comprising at least the antigen binding site, preferably an anti-CTLA-4, anti-CD25, anti-CCR8, anti-ICOS, anti-CD70, anti-GARP, anti-ILlRl, anti-CD39, anti-CCR4 or anti-CD 177 antibody; more preferably human or humanized antibody or whole antibody molecule, still more preferably whole human or humanized antibody molecule.

The protein or peptide of interest is advantageously fused to the N-terminus or the C-terminus of the IL-2 variant of the invention, directly or via a peptide spacer.

In preferred embodiments, the IL-2 variant or derived fusion protein as disclosed above, is complexed with an anti-IL-2 antibody. In some more preferred embodiments the anti-IL-2 antibody is an IL-2 antibody with pro-Treg function, i.e., which blocks IL-2RB/IL-2 interaction and induces structure modification on IL-2. Such antibodies are well-known in the art and include for example clone JES6-1A12 (rat IgG2A anti-mouse IL-2) and clone 5344.111 (mouse IgGl anti-human IL-2). These Abs have shown in vivo capacity to expand Tregs in pre-clinical models of diabetes (Tang Q et al, Immunity, 2008, 28, 687-97; Grinberg-Bleyer Y et al, J Exp Med, 2010, 207, 1871-8), allergy (Smaldini PL et al, Allergy, 2018, 73, 885-895), multiple sclerosis (Webster K.E. et al, J Exp Med, 2009, 206, 751-60), rheumatoid arthritis (Lee-S Y, et al, Immunology, 2012, 137, 305-16) and transplantation (Vokaer B, et al, Transplant Proc., 2012, 44, 2840-4). In some other more preferred embodiments the anti-IL-2 antibody is an IL-2 antibody with pro-Teff function. Such antibodies are well-known in the art and include for example, clone S4B6 (rat IgG2A anti-mouse IL-2) and clone Mab602 (mouse IgG2a anti -human IL-2). These Abs have shown in vivo better capacity than IL-2 to control tumor growth in melanoma (as single treatment or combined) (Boyman O et al, Science 2006, 311, 1921-27, Krieg et al, PNAS, 2010, 107, 11906-11, Caudana T et al, 2019, Cancer Immunol Res, 7, 443-457) and lymphoma (Newman RG et al, Blood, 2014, 123, 3045-55). The anti-IL-2 antibody is advantageously a human or humanized anti-IL-2 antibody, including humanized antibodies derived from the above mouse or rat monoclonal antibodies.

In preferred embodiments, the IL-2 variant or derived fusion protein as disclosed above, is complexed with an antibody against a Treg specific surface molecule as defined above, preferably a whole antibody, more preferable a whole human antibody.

In preferred embodiments, the IL-2 variant is linked to a CAR T-cell, in particular a patient's CAR T-cell.

The terms *"IL-2 variant"* as used herein encompasses the different forms of IL-2 variant disclosed herein, such as an IL-2 variant, modified or not, associated or not with at least an agent of interest in the form of a complex, conjugate or fusion protein as disclosed above.

The IL-2 variant according to the invention can be made by routine techniques in the art, in particular by expression of a recombinant DNA in a suitable cell system (eukaryotic or prokaryotic) and screened for activity (i.e. capable of preferentially stimulating T-regulatory cells) using the assays described herein or other similar assays.

### Polynucleotide, vector and host cell

The invention also relates to an isolated polynucleotide encoding an IL-2 variant according to the invention in an expressible form, and more particularly encoding the above-mentioned amino acid sequences.

Polynucleotides encoding an IL-2 variant of the invention in expressible form refers to a nucleic acid molecule which, upon expression in a cell or a cell-free system, results in a functional protein.

A polynucleotide according to the invention may in particular have at least 80% amino acid identity with a sequence selected from the group consisting of SEQ ID NO: 8, SEQ ID NO: 9, SEQ ID NO: 10, SEQ ID NO: 11, and SEQ ID NO: 12. Preferably, said IL-2 variant of the invention has at least 80%, 81%, 82%, 83%, 84%, 85%, 86%, 87%, 88%, 89%, 90%, 91%, 92%, 93%, 94%, 95%, 96%, 97%, 98% or 99% sequence identity with any one of said sequences, even more preferably at least 95%, 96%, 97%, 98% or 99% 98% or 99% identity with any one of said sequences.

The polynucleotide, either synthetic or recombinant, may be DNA, RNA or combination thereof, either single- and/or double-stranded. The polynucleotide is operably linked to at least one transcriptional regulatory sequence and, optionally to at least one translational regulatory sequence. Preferably the polynucleotide comprises a coding sequence which is optimized for the host in which the IL-2 variant is expressed.

The polynucleotide according to the invention is prepared by the conventional methods known in the art. For example, it is produced by amplification of a nucleic sequence by PCR or RT-PCR, by screening genomic DNA libraries by hybridization with a homologous probe, or else by total or partial chemical synthesis.

Another aspect of the invention is a recombinant vector comprising said polynucleotide. The recombinant vector is advantageously an expression vector capable of expressing said polynucleotide when delivered into a host cell such as prokaryotic or eukaryotic cell, for example mammalian or bacterial cell. Recombinant vectors include usual vectors used in genetic engineering and gene therapy including for example plasmids and viral vectors.

The recombinant vectors are constructed and introduced into host cells by the conventional recombinant DNA, genetic engineering and gene therapy techniques, which are known in the art.

Thus, a further aspect of the invention provides a host cell comprising said polynucleotide or recombinant vector.

In some embodiments, the host cell is a patient cell such as T cell, NK cell or CAR T-cell, which is modified by the polynucleotide or vector according to the invention, for use for adoptive T cell therapy.

The polynucleotide, vector, cell of the invention are useful for the production of the variants of the invention using well-known recombinant DNA techniques.

### Pharmaceutical composition and therapeutic use

The IL-2 variant, polynucleotide, vector and/or host cell according to the invention are used for treating immune disorders.

In the context of the present invention, the terms *"treat"* or *"treatment",* in particular when used in relation with a disease, denotes slowing down or stopping the development or progression of the disease, causing regression of the associated clinical symptoms or relieving the disease partially or completely. The term "treat" must also be understood as encompassing the reduction of number of relapses and/or the reduction of intensity of the relapses and/or the increase of the time separating two relapses of a disease according to the present invention, in particular of an inflammatory bowel disease.

In some embodiments, the IL-2 variant, polynucleotide, vector and/or host cell according to the invention are used to expand Tregs *ex vivo* or *in vivo* or to block IL-2-mediated overactivation of the immune system, for treating diseases associated with immune dysregulation where immunomodulation or immunosuppression is beneficial, including diseases associated with dysfunction or overactivity of the immune system.

In particular, the IL-2 variants capable of preferentially stimulating T-regulatory cells (IL-2 agonists variants) are used for treating immune disorders associated with dysfunction of the immune system such as allergic and auto-immune diseases and immune disorders associated with overactivity of the immune system such as acute or chronic inflammatory diseases, GVHD and graft-rejection.

For *ex vivo* therapy, a peripheral blood sample is collected from the patient; T cells are expanded in vitro using at least an IL-2 variant, polynucleotide and/or vector according to the invention, and the expanded Tregs are then re-injected into the patient. For *in vivo* therapy, the IL-2 variant, polynucleotide and/or vector according to the invention are administered to the patient and the Tregs are expanded *in vivo* in the patient.

In some embodiments, the IL-2 antagonist variants, polynucleotide, vector and/or cell according to the invention are used to inhibit Tregs by depriving them from IL-2 and thus allowing the stimulation of immune responses including B, NK, CD4+ or CD8+ T cells, DC, macrophages and others, for treating cancer or infectious diseases or increasing the immune response against vaccines, in particular vaccines for cancer or infectious diseases. In some preferred embodiments, the IL-2 antagonist variants, polynucleotide, vector and/or cell according to the invention are used to stimulate an anti-tumoral CD8+ T-cell response or a CD8+ T-cell response against a pathogen or a vaccine including a vaccine against cancer or infectious disease.

For *in vivo* therapy, the IL-2 antagonist variants, polynucleotide and/or vector according to the invention are administered to the patient and the Tregs are inhibited or eliminated *in vivo* in the patient, unleashing immune cells (B, NK, CD4+ or CD8+ T cells; DC; macrophages and others) from Treg suppression.

The present invention relates to a pharmaceutical composition comprising, as active substance, at least an IL-2 variant, polynucleotide, vector, and/or cell, according to the invention, and at least one pharmaceutically acceptable carrier.

The pharmaceutical composition is formulated for administration by a number of routes, including but not limited to oral, parenteral and local. The pharmaceutical carriers are those appropriate to the planned route of administration, which are well known in the art. The term *"pharmaceutically acceptable carrier"* is intended to denote a medium which is compatible with the body of the individual to whom said composition must be administered. It is, for example, a non-toxic solvent such as water.

Non-limitative examples of carriers suitable for use in the composition of the invention include uni- or multi-lamellar liposomes, ISCOMS, virosomes, viral pseudoparticles, saponin micelles, saccharides (poly(lactide-co-glycolide)) or gold microspheres, and nanoparticles.

The pharmaceutical composition comprises a therapeutically effective amount of the IL-2 variant, polynucleotide, vector and/or cell sufficient to show a positive medical response in the individual to whom it is administered. A positive medical response refers to the reduction of subsequent (preventive treatment) or established (therapeutic treatment) disease symptoms. The positive medical response comprises a partial or total inhibition of the symptoms of the disease. A positive medical response can be determined by measuring various objective parameters or criteria such as objective clinical signs of the disease and/or the increase of survival. A medical response to the composition according to the invention can be readily verified in appropriate animal models of the disease which are well-known in the art and illustrated in the examples of the present application.

The pharmaceutically effective dose depends upon the composition used, the route of administration, the type of mammal (human or animal) being treated, the physical characteristics of the specific mammal under consideration, concurrent medication, and other factors, that those skilled in the medical arts will recognize.

By *"therapeutic regimen"* is meant the pattern of treatment of an illness, e.g., the pattern of dosing used during therapy. A therapeutic regimen may include an induction regimen and a maintenance regimen. The phrase *"induction regimen"* or *"induction period"* refers to a therapeutic regimen (or the portion of a therapeutic regimen) that is used for the initial treatment of a disease. The general goal of an induction regimen is to provide a high level of drug to a patient during the initial period of a treatment regimen. An induction regimen may employ (in part or in whole) a *"loading regimen",* which may include administering a greater dose of the drug than a physician would employ during a maintenance regimen, administering a drug more frequently than a physician would administer the drug during a maintenance regimen, or both. The phrase *"maintenance regimen"* or *"maintenance period"* refers to a therapeutic regimen (or the portion of a therapeutic regimen) that is used for the maintenance of a patient during treatment of an illness, e.g., to keep the patient in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., pain, disease manifestation, etc.]).

In some embodiments, the pharmaceutical composition comprises another active agent wherein said active agent is a pharmaceutical agent or therapeutic capable of preventing, treating or ameliorating a disease in humans or animals. The active agent may be a protein including an antibody, an oligonucleotide including an antisense oligonucleotide, peptide nucleic acid (PNA), small interfering RNA, locked nucleic acids (LNA), phosphorodiamidate morpholino oligonucleotides (PMO) and decoy DNA molecule, a plasmid, an aptamer including DNA, RNA or peptide aptamer, a small or large chemical drug, or mixtures thereof. In particular, the active agent may be an antiinflammatory or immunomodulatory agent such as for example rapamycin, corticoids or biologicals. The active agent may also be an anticancer or anti-infectious agent or an antigen such as a tumor antigen or an antigen of a pathogen.

The invention provides also an IL-2 variant, polynucleotide, vector, cell or pharmaceutical composition according to the invention for use as a medicament.

The invention provides also an IL-2 variant, polynucleotide, vector, cell, or pharmaceutical composition according to the invention for use in the prevention or treatment of immune disorders.

In the context of the present invention, the term *"prevent"* denotes the reduction to a lesser degree of the risk or of the probability of occurrence of a given phenomenon, that is to say, in the present invention, an immune disorder. The term *"prevent"* must also be understood as encompassing the reduction to a lesser degree of the risk or of the probability of relapse of a disease according to the present invention, in particular of a chronic one.

In some embodiments, the disease associated with immune dysfunction or dysregulation is a disease where immunosuppression is beneficial such as with no limitations an allergic disease, an autoimmune disease, and diseases associated with overactivity of the immune system, such as with no limitations a chronic or acute inflammatory disease, graft-versus-host disease (GVHD) or graft rejection. Non-limiting examples of autoimmune diseases include: type 1 diabetes, rheumatoid arthritis, psoriasis and psoriatic arthritis, multiple sclerosis, Systemic lupus erythematosus (lupus), Inflammatory bowel disease such as Crohn's disease and ulcerative colitis, Addison's disease, Grave's disease, Sjogren's disease, alopecia areata, autoimmune thyroid disease such as Hashimoto's thyroiditis, myasthenia gravis, vasculitis including HCV-related vasculitis and systemic vasculitis, uveitis, myositis, pernicious anemia, celiac disease, Guillain-Barre Syndrome, chronic inflammatory demyelinating polyneuropathy, scleroderma, hemolytic anemia, glomerulonephritis, autoimmune encephalitis, fibromyalgia, aplastic anemia and others. Non-limiting examples of inflammatory and allergic diseases include: neuro-degenerative disorders such as Parkinson disease, chronic infections such as parasitic infection or disease like Trypanosoma cruzi infection, allergy such as asthma, atherosclerosis, chronic nephropathy, and others. The disease may be allograft rejection including transplant-rejection, graft-versus-host disease (GVHD) and spontaneous abortion

In some embodiments, the disease associated with immune dysregulation or dysfunction is a disease where inhibition of Tregs and/or stimulation of immune responses, in particular CD8+ T-cell immune responses, is beneficial such as with no limitations cancers and infectious diseases. The invention includes the treatment of infectious diseases and cancer and the increase of the immune response against vaccines, in particular vaccines for cancer or infectious diseases.

As used herein, the term "cancer" refers to any member of a class of diseases or disorders characterized by uncontrolled division of cells and the ability of these cells to invade other tissues, either by direct growth into adjacent tissue through invasion or by implantation into distant sites by metastasis. Metastasis is defined as the stage in which cancer cells are transported through the bloodstream or lymphatic system. The term cancer according to the present invention also comprises cancer metastases and relapse of cancer. Cancers are classified by the type of cell that the tumor resembles and, therefore, the tissue presumed to be the origin of the tumor. For example, carcinomas are malignant tumors derived from epithelial cells. This group represents the most common cancers, including the common forms of breast, prostate, lung, and colon cancer. Lymphomas and leukemias include malignant tumors derived from blood and bone marrow cells. Sarcomas are malignant tumors derived from connective tissue or mesenchymal cells. Mesotheliomas are tumors derived from the mesothelial cells lining the peritoneum and the pleura. Gliomas are tumors derived from glia, the most common type of brain cell. Germinomas are tumors derived from germ cells, normally found in the testicle and ovary. Choriocarcinomas are malignant tumors derived from the placenta.

As used herein, the term "cancer" refers to any cancer that may affect any one of the following tissues or organs: breast; liver; kidney; heart, mediastinum, pleura; floor of mouth; lip; salivary glands; tongue; gums; oral cavity; palate; tonsil; larynx; trachea; bronchus, lung; pharynx, hypopharynx, oropharynx, nasopharynx; esophagus; digestive organs such as stomach, intrahepatic bile ducts, biliary tract, pancreas, small intestine, colon; rectum; urinary organs such as bladder, gallbladder, ureter; rectosigmoid junction; anus, anal canal; skin; bone; joints, articular cartilage of limbs; eye and adnexa; brain; peripheral nerves, autonomic nervous system; spinal cord, cranial nerves, meninges; and various parts of the central nervous system; connective, subcutaneous and other soft tissues; retroperitoneum, peritoneum; adrenal gland; thyroid gland; endocrine glands and related structures; female genital organs such as ovary, uterus, cervix uteri; corpus uteri, vagina, vulva; male genital organs such as penis, testis and prostate gland; hematopoietic and reticuloendothelial systems; blood; lymph nodes; thymus.

A cancer according to the invention may be selected from the group consisting of leukemias; seminomas; melanomas; teratomas; lymphomas, in particular non-Hodgkin lymphoma; neuroblastomas; gliomas; adenocarcinoma; mesothelioma, in particular pleural mesothelioma, peritoneal mesothelioma, pericardial mesothelioma and end stage mesothelioma; rectal cancer; endometrial cancer; thyroid cancer, in particular papillary thyroid carcinoma, follicular thyroid carcinoma, medullary thyroid carcinoma, undifferentiated thyroid cancer, multiple endocrine neoplasia type 2 A, multiple endocrine neoplasia type 2B, familial medullary thyroid cancer, pheochromocytoma and paraganglioma; skin cancer, in particular malignant melanoma, basal cell carcinoma, squamous cell carcinoma, Kaposi's sarcoma, keratoacanthoma, moles, dysplastic nevi, lipoma, angioma and dermatofibroma; nervous system cancer; brain cancer, in particular astrocytoma, medulloblastoma, glioma, lower grade glioma, ependymoma, germinoma, glioblastoma multiform, oligodendroglioma, schwannoma, retinoblastoma, congenital tumors, spinal cord neurofibroma, glioma or sarcoma; skull cancer, in particular osteoma, hemangioma, granuloma, xanthoma or osteitis deformans; meninges cancer, in particular meningioma, meningiosarcoma or gliomatosis; head and neck cancer, in particular head and neck squamous cell carcinoma and oral cancer, in particular buccal cavity cancer, lip cancer, tongue cancer, mouth cancer or pharynx cancer; lymph node cancer; gastrointestinal cancer; liver cancer, in particular hepatoma, hepatocellular carcinoma, cholangiocarcinoma, hepatoblastoma, angiosarcoma, hepatocellular adenoma and hemangioma; colon cancer; stomach or gastric cancer; esophageal cancer, in particular squamous cell carcinoma, larynx, adenocarcinoma, leiomyosarcoma or lymphoma; colorectal cancer; intestinal cancer; small bowel or small intestines cancer, in particular adenocarcinoma lymphoma, carcinoid tumors, Kaposi's sarcoma, leiomyoma, hemangioma, lipoma, neurofibroma or fibroma; large bowel or large intestines cancer, in particular adenocarcinoma, tubular adenoma, villous adenoma, hamartoma or leiomyoma; pancreatic cancer, in particular ductal adenocarcinoma, insulinoma, glucagonoma, gastrinoma, carcinoid tumors or vipoma; ear, nose and throat (ENT) cancer; breast cancer, in particular HER2-enriched breast cancer, luminal A breast cancer, luminal B breast cancer and triple negative breast cancer; cancer of the uterus, in particular endometrial cancer, more particularly endometrial carcinomas, endometrial stromal sarcomas and malignant mixed Miillerian tumors, uterine sarcomas, leiomyosarcomas and gestational trophoblastic disease; ovarian cancer, in particular dysgerminoma, Granulosa-Theca cell tumors and Sertoli-Leydig cell tumors; cervical cancer; vaginal cancer, in particular squamous-cell vaginal carcinoma, vaginal adenocarcinoma, clear cell vaginal adenocarcinoma, vaginal germ cell tumors, vaginal sarcoma botryoides and vaginal melanoma; vulvar cancer, in particular squamous cell vulvar carcinoma, verrucous vulvar carcinoma, vulvar melanoma, basal cell vulvar carcinoma, Bartholin gland carcinoma, vulvar adenocarcinoma and erythroplasia of Queyrat; genitourinary tract cancer; kidney cancer, in particular clear renal cell carcinoma, chromophobe renal cell carcinoma, papillary renal cell carcinoma, adenocarcinoma, Wilms tumor, nephroblastoma, lymphoma or leukemia; adrenal cancer; bladder cancer; urethra cancer, in particular squamous cell carcinoma, transitional cell carcinoma or adenocarcinoma; prostate cancer, in particular adenocarcinoma or sarcoma; testis cancer, in particular seminoma, teratoma, embryonal carcinoma, teratocarcinoma, choriocarcinoma, sarcoma, interstitial cell carcinoma, fibroma, fibroadenoma, adenomatoid tumors or lipoma; lung cancer, in particular small cell lung carcinoma (SCLC), non-small cell lung carcinoma (NSCLC), including squamous cell lung carcinoma, lung adenocarcinoma (LUAD), and large cell lung carcinoma, bronchogenic carcinoma, alveolar carcinoma, bronchiolar carcinoma, bronchial adenoma, lung sarcoma, chondromatous hamartoma and pleural mesothelioma; sarcomas, in particular Askin's tumor, sarcoma botryoides, chondrosarcoma, Ewing's sarcoma, malignant hemangioendothelioma, malignant schwannoma, osteosarcoma and soft tissue sarcomas; soft tissue sarcomas, in particular alveolar soft part sarcoma, angiosarcoma, cystosarcoma phyllodes, dermatofibrosarcoma protuberans, desmoid tumor, desmoplastic small round cell tumor, epithelioid sarcoma, extraskeletal chondrosarcoma, extraskeletal osteosarcoma, fibrosarcoma, gastrointestinal stromal tumor (GIST), hemangiopericytoma, hemangiosarcoma, Kaposi's sarcoma, leiomyosarcoma, liposarcoma, lymphangio sarcoma, lymphosarcoma, malignant peripheral nerve sheath tumor (MPNST), neurofibrosarcoma, plexiform fibrohistiocytic tumor, rhabdomyosarcoma, synovial sarcoma and undifferentiated pleomorphic sarcoma; cardiac cancer, in particular angiosarcoma, fibrosarcoma, rhabdomyosarcoma, liposarcoma, myxoma, rhabdomyoma, fibroma, lipoma and teratoma; bone cancer, in particular osteogenic sarcoma, osteosarcoma, fibrosarcoma, malignant fibrous histiocytoma, chondrosarcoma, Ewing's sarcoma, malignant lymphoma and reticulum cell sarcoma, multiple myeloma, malignant giant cell tumor chordoma, osteochronfroma, osteocartilaginous exostoses, benign chondroma, chondroblastoma, chondromyxoid fibroma, osteoid osteoma and giant cell tumors; hematologic and lymphoid cancer; blood cancer, in particular acute myeloid leukemia, chronic myeloid leukemia, acute lymphoblastic leukemia, chronic lymphocytic leukemia, myeloproliferative diseases, multiple myeloma and myelodysplasia syndrome; Hodgkin's disease; non-Hodgkin's, and the metastases thereof.

Infectious diseases include viral, bacterial, fungal and parasitic diseases such as for example HIV/AIDS, Covid-19, viral hepatitis such as Hepatitis A, Hepatitis B, Hepatitis C, measles, malaria, and Chagas disease.

The invention provides also a method for treating a disease associated with immune dysfunction or dysregulation, comprising administering a therapeutically effective amount of an IL-2 variant according to the invention or of a pharmaceutical composition according to the invention to an individual in need thereof.

An IL-2 variant or pharmaceutical composition of the present invention is generally administered according to known procedures, at dosages and for periods of time effective to induce a beneficial effect in the individual. The administration may be by injection or by oral, sublingual, intranasal, rectal or vaginal administration, inhalation, or transdermal application. The injection may be subcutaneous, intramuscular, intravenous, intraperitoneal, intradermal or else.

An IL-2 variant or pharmaceutical composition of the present invention is advantageously used in combination with another therapy, in particular immunotherapy such as CAR-T cell therapy; therapy with an immunomodulatory agent, in particular an immunomodulatory monoclonal antibody or functional derivative thereof; therapy with anticancer or anti-infectious agents including therapeutic agents and vaccines against cancer and infectious diseases. The combined therapies may be separate, simultaneous, and/or sequential.

In some embodiments, an IL-2 variant or pharmaceutical composition of the present invention is used for the prevention or treatment of humans.

In some embodiments, an IL-2 variant or pharmaceutical composition of the present invention is used for the treatment of animals.

In some embodiments, the IL-2 variant, polynucleotide, vector, cell, and/or pharmaceutical composition according to the invention is administered in combination with additional cancer therapies. In particular, IL-2 variant, polynucleotide, vector, cell and/or pharmaceutical composition of the invention may be administered in combination with targeted therapy, immunotherapy such as immune checkpoint therapy and immune checkpoint inhibitor, co- stimulatory antibodies, chemotherapy and/or radiotherapy.

Accordingly, an IL-2 variant or pharmaceutical composition of the present invention may be administered to the individual in need thereof in combination with at least one immune check-point inhibitor; preferably anti-PD-1 and/or anti-PDL-1.

Immune checkpoint therapy such as checkpoint inhibitors include, but are not limited to programmed death- 1 (PD-1) inhibitors, programmed death ligand- 1 (PD-L1) inhibitors, programmed death ligand-2 (PD-L2) inhibitors, lymphocyte-activation gene 3 (LAG-3) inhibitors, T-cell immunoglobulin and mucin-domain containing protein 3 (TIM-3) inhibitors, T cell immunoreceptor with Ig and ITIM domains (TIGIT) inhibitors, B- and T-lymphocyte attenuator (BTLA) inhibitors, V-domain Ig suppressor of T-cell activation (VISTA) inhibitors, cytotoxic T-lymphocyte-associated protein 4 (CTLA-4) inhibitors, Indoleamine 2,3-dioxygenase (IDO) inhibitors, killer immunoglobulin-like receptors (KIR) inhibitors, KIR2L3 inhibitors, KIR3DL2 inhibitors and carcinoembryonic antigen-related cell adhesion molecule 1 (CEACAM-1) inhibitors. In particular, checkpoint inhibitors include antibodies anti-PD-1, anti-PD-L1, anti-CTLA-4, anti-TIM-3, anti-LAG3. Co stimulatory antibodies deliver positive signals through immune-regulatory receptors including but not limited to ICOS, CD137, CD27, OX-40 and GITR.

Examples of anti-PD-1 antibodies include, but are not limited to, nivolumab, cemiplimab (REGN2810 or REGN-2810), tislelizumab (BGB-A317), spartalizumab (PDR001 or PDR-001), ABB V- 181, JNJ-63723283, BI 754091, MAG012, TSR-042, AGEN2034, pidilizumab, nivolumab (ONO-4538, BMS-936558, MDX1106, GTPL7335 or Opdivo), pembrolizumab (MK-3475, MK03475, lambrolizumab, SCH-900475 or Keytruda) and antibodies described in International patent applications WO2004004771, WO2004056875, WO2006121168, WO2008156712, WO2009014708, WO2009114335, WO2013043569 and WO2014047350.

Examples of anti-PD-L1 antibodies include, but are not limited to, LY3300054, atezolizumab, durvalumab and avelumab.

Examples of anti-CTLA-4 antibodies include, but are not limited to, ipilimumab (see, e.g., US patents US6,984,720 and US8,017,114), tremelimumab (see, e.g., US patents US7, 109,003 and US8, 143,379), single chain anti-CTLA4 antibodies (see, e.g., International patent applications WO1997020574 and WO2007123737) and antibodies described in US patent US8,491,895.

Example of anti- VISTA antibodies are described in US patent application US20130177557.

Example of inhibitors of the LAG3 receptor are described in US patent US5,773,578.

An example of KIR inhibitor is IPH4102 targeting KIR3DL2.

Targeted therapies are drugs designed to interfere with specific molecules necessary for tumor growth and progression. For example, therapeutic monoclonal antibodies target specific antigens found on the cell surface, such as transmembrane receptors or extracellular growth factors. In some cases, monoclonal antibodies are conjugated to radioisotopes or toxins to allow specific delivery of these cytotoxic agents to the intended cancer cell target. Small molecules can penetrate the cell membrane to interact with targets inside a cell. Small molecules are usually designed to interfere with the enzymatic activity of the target protein such as for example proteasome inhibitor, tyrosine kinase or cyclin-dependent kinase inhibitor, histone deacetylase inhibitor. Targeted therapy may also use cytokines. Examples of such targeted therapy include with no limitations: Ado-trastuzumab emtansine (HER2), Afatinib (EGFR (HER1/ERBB1), HER2), Aldesleukin (Proleukin), alectinib (ALK), Alemtuzumab (CD52), axitinib (kit, PDGFRbeta, VEGFR1/2/3), Belimumab (BAFF), Belinostat (HDAC), Bevacizumab (VEGF ligand), Blinatumomab (CD19/CD3), bortezomib (proteasome), Brentuximab vedotin (CD30), bosutinib (ABL), brigatinib (ALK), cabozantinib (FLT3, KIT, MET, RET, VEGFR2), Canakinumab (IL-1 beta), carfilzomib (proteasome), ceritinib (ALK), Cetuximab (EGFR), cofimetinib (MEK), Crizotinib (ALK, MET, ROS1), Dabrafenib (BRAF), Daratumumab (CD38), Dasatinib (ABL), Denosumab (RANKL), Dinutuximab (B4GALNT1 (GD2)), Elotuzumab (SLAMF7), Enasidenib (IDH2), Erlotinib (EGFR), Everolimus (mTOR), Gefitinib (EGFR), Ibritumomab tiuxetan (CD20), Sonidegib (Smoothened), Sipuleucel-T, Siltuximab (IL-6), Sorafenib (VEGFR, PDGFR, KIT, RAF),(Tocilizumab (IL-6R), Temsirolimus (mTOR), Tofacitinib (JAK3), Trametinib (MEK), Tositumomab (CD20), Trastuzumab (HER2), Vandetanib (EGFR), Vemurafenib (BRAF), Venetoclax (BCL2), Vismodegib (PTCH, Smoothened), Vorinostat (HDAC), Ziv-aflibercept (PIGF, VEGFA/B).

In some embodiments, the IL-2 variant, polynucleotide, vector, cell and/or pharmaceutical composition of the invention is administered to the patient in combination with chemotherapy. As used herein, the term "chemotherapy" has its general meaning in the art and refers to the treatment that consists in administering to the patient a chemotherapeutic agent. Chemotherapeutic agents include, but are not limited to alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB 1-TM1); eleutherobin; pancratistatin; a sarcodictyin; spongistatin; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g. , calicheamicin, especially calicheamicin gammall and calicheamicin omegall ; dynemicin, including dynemicin A; bisphosphonates, such as clodronate; an esperamicin; as well as neocarzinostatin chromophore and related chromoprotein enediyne antiobiotic chromophores, aclacinomysins, actinomycin, authramycin, azaserine, bleomycins, cactinomycin, carabicin, caminomycin, carzinophilin, chromomycinis, dactinomycin, daunorubicin, detombicin, 6-diazo-5-oxo-L-norleucine, doxorubicin (including morpholino-doxorubicin, cyanomorpholino-doxombicin, 2-pyrrolino-doxorubicin and deoxy doxorubicin), epirubicin, esombicin, idarubicin, marcellomycin, mitomycins such as mitomycin C, mycophenolic acid, nogalamycin, olivomycins, peplomycin, potfiromycin, puromycin, quelamycin, rodombicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; methylhydrazine derivatives including N-methylhydrazine (MIH) and procarbazine; PSK polysaccharide complex); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., paclitaxel and doxetaxel; chlorambucil; gemcitabine; 6-thioguanine; mercaptopurine; methotrexate; platinum coordination complexes such as cisplatin, oxaliplatin and carboplatin; vinblastine; platinum; etoposide (VP- 16); ifosfamide; mitoxantrone; vincristine; vinorelbine; novantrone; teniposide; edatrexate; daunomycin; aminopterin; xeloda; ibandronate; irinotecan (e.g., CPT-1 1); topoisomerase inhibitor RFS 2000; difluoromethylomithine (DMFO); retinoids such as retinoic acid; capecitabine; anthracyclines, nitrosoureas, antimetabolites, epipodophylotoxins, enzymes such as L-asparaginase; anthracenediones; hormones and antagonists including adrenocortico steroid antagonists such as prednisone and equivalents, dexamethasone and aminoglutethimide; progestin such as hydroxyprogesterone caproate, medroxyprogesterone acetate and megestrol acetate; estrogen such as diethylstilbestrol and ethinyl estradiol equivalents; antiestrogen such as tamoxifen; androgens including testosterone propionate and fluoxymesterone/equivalents; antiandrogens such as flutamide, gonadotropin-releasing hormone analogs and leuprolide; and non-steroidal antiandrogens such as flutamide; and pharmaceutically acceptable salts, acids or derivatives of any of the above.

In some embodiments, the IL-2 variant, polynucleotide, vector, cell and/or pharmaceutical composition of the invention is administered to the patient in combination with radiotherapy. Suitable examples of radiation therapies include, but are not limited to external beam radiotherapy (such as superficial X-rays therapy, orthovoltage X-rays therapy, megavoltage X-rays therapy, radiosurgery, stereotactic radiation therapy, Fractionated stereotactic radiation therapy, cobalt therapy, electron therapy, fast neutron therapy, neutron-capture therapy, proton therapy, intensity modulated radiation therapy (IMRT), 3-dimensional conformal radiation therapy (3D-CRT) and the like); brachytherapy; unsealed source radiotherapy; tomotherapy; and the like. Gamma rays are another form of photons used in radiotherapy. Gamma rays are produced spontaneously as certain elements (such as radium, uranium, and cobalt 60) release radiation as they decompose, or decay. In some embodiments, radiotherapy may be proton radiotherapy or proton minibeam radiation therapy. Proton radiotherapy is an ultra-precise form of radiotherapy that uses proton beams (Prezado Y, Jouvion G, Guardiola C, Gonzalez W, Juchaux M, Bergs J, Nauraye C, Labiod D, De Marzi L, Pouzoulet F, Patriarca A, Dendale R. Tumor Control in RG2 Glioma-Bearing Rats: A Comparison Between Proton Minibeam Therapy and Standard Proton Therapy. Int J Radiat Oncol Biol Phys. 2019 Jun 1;104(2):266-271. doi: 10.1016/j.ijrobp.2019.01.080; Prezado Y, Jouvion G, Patriarca A, Nauraye C, Guardiola C, Juchaux M, Lamirault C, Labiod D, Jourdain L, Sebrie C, Dendale R, Gonzalez W, Pouzoulet F. Proton minibeam radiation therapy widens the therapeutic index for high-grade gliomas. Sci Rep. 2018 Nov 7;8(1):16479. doi: 10.1038/s41598-018-34796-8). Radiotherapy may also be FLASH radiotherapy (FLASH-RT) or FLASH proton irradiation. FLASH radiotherapy involves the ultra-fast delivery of radiation treatment at dose rates several orders of magnitude greater than those currently in routine clinical practice (ultra-high dose rate) (Favaudon V, Fouillade C, Vozenin MC. The radiotherapy FLASH to save healthy tissues. Med Sci (Paris) 2015 ; 31 : 121-123. DOI: 10.105 l/medsci/20153102002); Patriarca A., Fouillade C. M., Martin F., Pouzoulet F., Nauraye C., et al. Experimental set-up for FLASH proton irradiation of small animals using a clinical system. Int J Radiat Oncol Biol Phys, 102 (2018), pp. 619-626. doi: 10.1016/j.ijrobp.2018.06.403. Epub 2018 Jul 11).

The invention will be further illustrated by the following figures and examples. However, these examples and figures should not be interpreted in any way as limiting the scope of the present invention.

### EXAMPLES: IN VITRO EVALUATION OF THE BIOLOGICAL ACTIVITY OF IL-2 VARIANTS OF THE INVENTION

It was evaluated whether different IL-2 variants according to the invention showed different affinities/selectivity for the T cell populations expressing either the IL-2R of intermediate affinity (present in CD4+ or CD8+ T-effector cell) or the IL-2R of high affinity (present in Tregs).

Therefore, PBMCs from healthy donors were cultured in 96-well plates in 75 µL/well in SVF-free RPMI medium (GIBCO, France) containing different concentrations of:
- Proleukine (NOVARTIS, from 0,0001 nM to 1000 nM)) ; or
- FcV15 (V15 being a human IL-2 variant according to the invention consisting in the amino acid substitution H16R and one amino acid deletion at a position selected from S4, S5 or S6, the identified positions being determined by alignment with the sequence set forth as SEQ ID NO: 1 (SEQ ID NO: 2)); (from 0,00005 nM to 500 nM, corresponding to Proleukin concentration of 0,0001 nM to 1000 nM); or
- FcV17 (V17 being a human IL-2 variant according to the invention consisting in the amino acid substitutions H16R and L19R and at least one, and in particular one, amino acid deletion at a position selected from S4, S5 or S6, the identified positions being determined by alignment with the sequence set forth as SEQ ID NO: 1 (SEQ ID NO: 3)); (from 0,00005 nM to 500 nM, corresponding to Proleukin concentration of 0,0001 nM to 1000 nM); or
- FcV9 (V9 being a human IL-2 variant according to the invention consisting in the amino acid substitutions H16R and V91K and at least one, and in particular one, amino acid deletion at a position selected from S4, S5 or S6, the identified positions being determined by alignment with the sequence set forth as SEQ ID NO: 1 (SEQ ID NO: 4)); (from 0,00005 nM to 500 nM, corresponding to Proleukin concentration of 0,0001 nM to 1000 nM); or
- FcV18 (V18 being a human IL-2 variant according to the invention consisting in the amino acid substitutions L19R and V91K and at least one, and in particular one, amino acid deletion at a position selected from S4, S5 or S6, the identified positions being determined by alignment with the sequence set forth as SEQ ID NO: 1 (SEQ ID NO: 5)); (from 0,00005 nM to 500 nM, corresponding to Proleukin concentration of 0,0001 nM to 1000 nM); or
- FcV10 (V10 being a human IL-2 variant according to the invention consisting in the amino acid substitutions H16R, L19R and V91K and at least one, and in particular one, amino acid deletion at a position selected from S4, S5 or S6, the identified positions being determined by alignment with the sequence set forth as SEQ ID NO: 1 (SEQ ID NO: 6)) (from 0,00005 nM to 500 nM, corresponding to Proleukin concentration of 0,0001 nM to 1000 nM).

For competition experiments with WT IL-2, PBMCs from healthy donors were cultured in 96-well plates in 75 µL/well in SVF-free RPMI medium (GIBCO, France) containing different concentrations (from 0,0001 nM to 1000 nM) of Proleukine (NOVARTIS) and a fixed concentration of 500 nM of Fc-V9 or Fc-V10.

The tested variants were produced and tested in the form of Fc-[Variant]s, the molecules being composed of a silent Fc moiety fused to 2 identical IL-2 variants as defined above through each N-terminal of the variant, using a (GGGGS)3 linker (SEQ ID NO: 7).

Phosphorylation of STATS (pSTAT5) in human Tregs (IL-2Raby), CD4+ Teff cells (IL-2Rby) and CD8+ T cells (IL-2Rbg) was measured by flow cytometry, as follows. After 15 min of stimulation, cultures were fixed with 200 uL/well of PBS/2% paraformaldehyde for 10 min at room temperature. After washing with PBS/0.2% BSA, cells were permeabilized with 100 uL/well of ice-cold methanol for 10 min on ice. Cells were then washed with PBS/0.2% BSA and stained with anti-CD3 PE-Cy7 (clone UCHT1; 1:200; BD BIOSCIENCES), anti-CD4 PE-CF594 (clone RPA-T4 1:50; BD BIOSCIENCES) or BUV395 (clone RPA-T4 1:100; BD BIOSCIENCES), anti-CD25 PE (clone M-A251; 1:5; BD BIOSCIENCES), anti-Foxp3 Alexa488 (clone 236A/E7; 1:20; BD BIOSCIENCES) and anti-pSTAT5 Alexa647 (clone 47/Stat5(pY694); 1:20; BD BIOSCIENCES) for 45 min at 4°C. Cells were acquired on a Fortessa flow cytometer and analyzed with FlowJo software.

The results show that human IL-2 variants V15 (Figure 1), V17 (Figure 2) and V18 (Figure 4) selectively induce STAT-5 phosphorylation in Tregs but not in Tconv or CD8+ T cells in comparison with human IL-2 (Proleukine). Human IL-2 variants V9 (Figure 3) and V10 (Figure 5) do not stimulate Tregs or Tconv or CD8+ cells at the evaluated doses. Moreover, IL-2V9 (Figure 6) and IL-2V10 (Figure 7) compete with WT IL-2 signaling in Tregs, underlying their antagonistic function.

### Description of the Sequences

SEQ ID NO: 1
   **Human IL-2 WT amino acide sequence**
**SEQ ID NO: 2**
   **IL-2 variant V15 amino acid sequence** 
APTSSTKKTQLQLERLLLDLQMILNGINNYKNPKLTRMLTFKFYMPKKATELKHLQCLEEE
LKPLEEVLNLAQSKNFHLRPRDLISNINVIVLELKGSETTFMCEYADETATIVEFLNRWITFC
QSIISTLT
**SEQ ID NO: 3**
   **IL-2 variant V17 amino acid sequence** 
APTSSTKKTQLQLERLLRDLQMILNGINNYKNPKLTRMLTFKFYMPKKATELKHLQCLEE
ELKPLEEVLNLAQSKNFHLRPRDLISNINVIVLELKGSETTFMCEYADETATIVEFLNRWITF
CQSIISTLT
**SEQ ID NO: 4**
   **IL-2 variant V9 amino acid sequence** 
APTSSTKKTQLQLERLLLDLQMILNGINNYKNPKLTRMLTFKFYMPKKATELKHLQCLEEE
LKPLEEVLNLAQSKNFHLRPRDLISNINKIVLELKGSETTFMCEYADETATIVEFLNRWITFC
QSIISTLT
SEQ ID NO: 5
   **IL-2 variant V18 amino acid sequence** 
APTSSTKKTQLQLEHLLRDLQMILNGINNYKNPKLTRMLTFKFYMPKKATELKHLQCLEE
ELKPLEEVLNLAQSKNFHLRPRDLISNINKIVLELKGSETTFMCEYADETATIVEFLNRWITF
CQSIISTLT
**SEQ ID NO: 6**
   **IL-2 variant V10 amino acid sequence** 
APTSSTKKTQLQLERLLRDLQMILNGINNYKNPKLTRMLTFKFYMPKKATELKHLQCLEE
ELKPLEEVLNLAQSKNFHLRPRDLISNINKIVLELKGSETTFMCEYADETATIVEFLNRWITF
CQSIISTLT
**SEQ ID NO: 7**
   **Linker**
   GGGGSGGGGSGGGGS
**SEQ ID NO: 8**
   **IL-2 variant V15 nucleic acid sequence** 
gcgcccaccagtagcaccaaaaagacccagctgcaattagagcggctgttgctggatctgcaaatgatactgaacggcattaaca
actataaaaatcccaagctgacgaggatgctgacattcaagttctacatgcccaagaaggctactgaactgaaacacctgcaatgct
tggaggaagagctgaagccactggaggaagtgctcaatttagcacagagcaagaactttcaccttcggccgcgggatctgatctct
aatattaatgtcatagtcctggagttaaaggggagtgaaactacatttatgtgcgaatatgccgacgaaacagcaactatagtcgagt
ttctgaacaggtggatcacattttgccagtctatcatctctactctgact
**SEQ ID NO: 9**
   **IL-2 variant V17 nucleic acid sequence** 
gccccaacttcttcaaccaagaagactcagctccagttggagcgactgctacgtgacctccagatgattctgaatgggatcaataac
tataaaaatccaaagctgactaggatgcttacatttaagttttatatgcctaagaaggcgaccgagctcaagcatttacagtgtctgga
agaggagctgaagcctttggaggaagtcttgaatctggctcaatccaagaattttcacctgagacccagggatttgatttcaaacatc
aacgtgattgtgttagagctgaagggttctgagaccacatttatgtgtgagtacgctgacgaaaccgctaccattgtggaatttctgaa
cagatggattacattctgtcagtcaatcatcagcaccctgacc
**SEQ ID NO: 10**
   **IL-2 variant V9 nucleic acid sequence** 
gcacccaccagttcgaccaaaaagacgcagctgcaattagagcggctgttactggatctgcaaatgatactgaacggcattaacaa
ctataaaaatcccaagctgacaaggatgctgacattcaagttctacatgcccaagaaggccactgaactgaaacacctgcaatgctt
ggaggaagagctgaagccactagaggaggtgctcaatttagcacagtcgaagaactttcaccttcggccgcgggacctgatctct
aatattaataaaatagtcctggagctgaaggggagcgaaactacatttatgtgcgagtatgccgatgaaacagcaactatagtcgag
tttctgaacaggtggatcacattttgccagtctatcatctctactctgact
**SEQ ID NO: 11**
   **IL-2 variant V18 nucleic acid sequence** 
gcgccaacctcaagtacaaaaaagacacagctgcaactggagcatctgcttcgagacctgcaaatgatcctgaatggcattaaca
actataaaaacccaaaattaacaaggatgctgactttcaagttttacatgcctaagaaggccacagaactgaaacatctgcaatgtct
ggaggaggagttgaagccactggaggaggtcctaaacctggcacagtctaagaattttcatctgaggccgcgggatctaatctcc
aatataaataaaatagtccttgagctgaaaggaagcgagacaacttttatgtgcgaatatgctgacgagaccgctaccatagtcgag
tttctgaatagatggattactttctgccaaagtataatttcaaccctgacc
**SEQ ID NO: 12**
   **IL-2 variant V10 nucleic acid sequence**

gcgcctaccagcagtaccaagaaaacccagctgcaactggagaggctgctgcgggacctgcaaatgattctgaacggtatcaac
aactacaaaaatcctaaacttacacgtatgctgacctttaaattttacatgcctaaaaaggccaccgagctcaagcatctgcaatgctt
ggaggaagagctcaagccgctggaagaggtgctgaatttggcccaatccaagaacttccacctgcggcctcgcgacctgatcag
caacattaataaaatcgtgttggaactgaagggcagtgagactacgtttatgtgtgagtacgctgacgagaccgcgaccattgtcga
gtttctgaatagatggatcacattttgtcagtccatcatttcgacgctgact

## Claims

1. An interleukin-2 variant which comprises:
(i) the amino acid substitution H16R; or
(ii) the amino acid substitution H16R and one or two amino acid substitution(s) selected from the group consisting of L19R and V91K; or
(iii) the amino acid substitution L19R and the amino acid substitution V91K;
the identified positions being determined by alignment with the sequence set forth as SEQ ID NO: 1,
the IL-2 variant comprising at most 10 substitutions compared with sequence SEQ ID NO: 1.

2. The interleukin-2 variant according to claim 1, which is selected from the group consisting of:
- a variant comprising the amino acid substitution H16R; or
- a variant comprising the amino acid substitutions H16R and L19R; or
- a variant comprising the amino acid substitutions H16R and V91K; or
- a variant comprising the amino acid substitutions L19R and V91K; or
- a variant comprising the amino acid substitutions H16R, L19R and V91K;
the identified positions being determined by alignment with the sequence set forth as SEQ ID NO: 1.

3. The interleukin-2 variant according to claim 1 or 2, which:
- does not comprise any substitution at at least one of the positions selected from the group consisting of positions 9, 12, 23, 26, 31, 87 and 95, and in particular does not comprise any substitution at any of positions 9, 12, 23, 26, 31, 87 and 95;
- more particularly does not comprise any substitution at at least one of the positions 9, 12, 23, 26, 31, 49, 52, 81, 84, 87, 95, 119, 123, 127, 131 and 132, and in particular does not comprise any substitution at any of positions 9, 12, 23, 26, 31, 49, 52, 81, 84, 87, 95, 119, 123, 127, 131 and 132;
the identified positions being determined by alignment with the sequence set forth as SEQ ID NO: 1.

4. The interleukin-2 variant according to any one of claims 1 to 3, comprising at least one, and in particular one, amino acid deletion at a position selected from S4, S5 or S6,
the indicated positions being determined by alignment with the sequence set forth as SEQ ID NO: 1.

5. The interleukin-2 variant according to any one of claims 1 to 4, which is selected from the group consisting of:
- a variant consisting in the amino acid substitution H16R and at least one, and in particular one, amino acid deletion at a position selected from S4, S5 or S6; or
- a variant consisting in the amino acid substitutions H16R and L19R and at least one, and in particular one, amino acid deletion at a position selected from S4, S5 or S6; or
- a variant consisting in the amino acid substitutions H16R and V91K and at least one, and in particular one, amino acid deletion at a position selected from S4, S5 or S6; or
- a variant consisting in the amino acid substitutions L19R and V91K and at least one, and in particular one, amino acid deletion at a position selected from S4, S5 or S6; or
- a variant consisting in the amino acid substitutions H16R, L19R and V91K and at least one, and in particular one, amino acid deletion at a position selected from S4, S5 or S6;
the identified positions being determined by alignment with the sequence set forth as SEQ ID NO: 1.

6. The interleukin-2 variant according to any one of claims 1 to 5, which is a human IL-2 variant.

7. The interleukin-2 variant according to any one of claims 1 to 6, which is an IL-2 agonist, the interleukin-2 variant being more particularly selected from the group consisting of:
- a variant comprising the amino acid substitution H16R, and in particular a variant consisting in the amino acid substitution H16R and at least one, and in particular one, amino acid deletion at a position selected from S4, S5 or S6;
- a variant comprising the amino acid substitutions H16R and L19R, and in particular a variant consisting in the amino acid substitutions H16R and L19R and at least one, and in particular one, amino acid deletion at a position selected from S4, S5 or S6; and
- a variant comprising the amino acid substitutions L19R and V91K, and in particular a variant consisting in the amino acid substitutions L19R and V91K and at least one, and in particular one, amino acid deletion at a position selected from S4, S5 or S6;
the identified positions being determined by alignment with the sequence set forth as SEQ ID NO: 1.

8. The interleukin-2 variant according to claim any one of claims 1 to 7, which stimulates T regulatory cells.

9. The interleukin-2 variant according to any one of claims 1 to 6, which is an IL-2 antagonist, the interleukin-2 variant being more particularly selected from the group consisting of:
- a variant comprising the amino acid substitutions H16R and V91K, and in particular a variant consisting in the amino acid substitutions H16R and V91K and at least one, and in particular one, amino acid deletion at a position selected from S4, S5 or S6; and
- a variant comprising the amino acid substitutions H16R, L19R and V91K, and in particular a variant consisting in the amino acid substitutions H16R, L19R and V91K and at least one, and in particular one, amino acid deletion at a position selected from S4, S5 or S6;
the identified positions being determined by alignment with the sequence set forth as SEQ ID NO: 1.

10. An isolated polynucleotide encoding an interleukin-2 variant according to any one of claims 1 to 9.

11. A recombinant vector comprising at least one polynucleotide according to claim 10.

12. A host cell, in particular a non-human host cell, comprising at least one polynucleotide according to claim 10 and/or at least one recombinant vector according to claim 11.

13. A pharmaceutical composition comprising, in a pharmaceutically acceptable carrier, an interleukin-2 variant according to any one of claims 1 to 9.

14. The pharmaceutical composition according to claim 10, or the interleukin-2 variant according to any one of claims 1 to 5 and 8, for use in the prevention or treatment of cancer in an individual in need thereof.

15. The pharmaceutical composition or the interleukin-2 variant for use according to claim 14, wherein the individual is a human being.

16. The pharmaceutical composition or interleukin-2 variant for use according to claim 14 or 15, wherein the cancer is selected from the group consisting of leukemias; seminomas; melanomas; teratomas; lymphomas, in particular non-Hodgkin lymphoma; neuroblastomas; gliomas; adenocarcinoma; mesothelioma, in particular pleural mesothelioma, peritoneal mesothelioma, pericardial mesothelioma and end stage mesothelioma; rectal cancer; endometrial cancer; thyroid cancer, in particular papillary thyroid carcinoma, follicular thyroid carcinoma, medullary thyroid carcinoma, undifferentiated thyroid cancer, multiple endocrine neoplasia type 2 A, multiple endocrine neoplasia type 2B, familial medullary thyroid cancer, pheochromocytoma and paraganglioma; skin cancer, in particular malignant melanoma, basal cell carcinoma, squamous cell carcinoma, Kaposi's sarcoma, keratoacanthoma, moles, dysplastic nevi, lipoma, angioma and dermatofibroma; nervous system cancer; brain cancer, in particular astrocytoma, medulloblastoma, glioma, lower grade glioma, ependymoma, germinoma, glioblastoma multiform, oligodendroglioma, schwannoma, retinoblastoma, congenital tumors, spinal cord neurofibroma, glioma or sarcoma; skull cancer, in particular osteoma, hemangioma, granuloma, xanthoma or osteitis deformans; meninges cancer, in particular meningioma, meningiosarcoma or gliomatosis; head and neck cancer, in particular head and neck squamous cell carcinoma and oral cancer, in particular buccal cavity cancer, lip cancer, tongue cancer, mouth cancer or pharynx cancer; lymph node cancer; gastrointestinal cancer; liver cancer, in particular hepatoma, hepatocellular carcinoma, cholangiocarcinoma, hepatoblastoma, angiosarcoma, hepatocellular adenoma and hemangioma; colon cancer; stomach or gastric cancer; esophageal cancer, in particular squamous cell carcinoma, larynx, adenocarcinoma, leiomyosarcoma or lymphoma; colorectal cancer; intestinal cancer; small bowel or small intestines cancer, in particular adenocarcinoma lymphoma, carcinoid tumors, Kaposi's sarcoma, leiomyoma, hemangioma, lipoma, neurofibroma or fibroma; large bowel or large intestines cancer, in particular adenocarcinoma, tubular adenoma, villous adenoma, hamartoma or leiomyoma; pancreatic cancer, in particular ductal adenocarcinoma, insulinoma, glucagonoma, gastrinoma, carcinoid tumors or vipoma; ear, nose and throat (ENT) cancer; breast cancer, in particular HER2-enriched breast cancer, luminal A breast cancer, luminal B breast cancer and triple negative breast cancer; cancer of the uterus, in particular endometrial cancer, more particularly endometrial carcinomas, endometrial stromal sarcomas and malignant mixed Miillerian tumors, uterine sarcomas, leiomyosarcomas and gestational trophoblastic disease; ovarian cancer, in particular dysgerminoma, Granulosa-Theca cell tumors and Sertoli-Leydig cell tumors; cervical cancer; vaginal cancer, in particular squamous-cell vaginal carcinoma, vaginal adenocarcinoma, clear cell vaginal adenocarcinoma, vaginal germ cell tumors, vaginal sarcoma botryoides and vaginal melanoma; vulvar cancer, in particular squamous cell vulvar carcinoma, verrucous vulvar carcinoma, vulvar melanoma, basal cell vulvar carcinoma, Bartholin gland carcinoma, vulvar adenocarcinoma and erythroplasia of Queyrat; genitourinary tract cancer; kidney cancer, in particular clear renal cell carcinoma, chromophobe renal cell carcinoma, papillary renal cell carcinoma, adenocarcinoma, Wilms tumor, nephroblastoma, lymphoma or leukemia; adrenal cancer; bladder cancer; urethra cancer, in particular squamous cell carcinoma, transitional cell carcinoma or adenocarcinoma; prostate cancer, in particular adenocarcinoma or sarcoma; testis cancer, in particular seminoma, teratoma, embryonal carcinoma, teratocarcinoma, choriocarcinoma, sarcoma, interstitial cell carcinoma, fibroma, fibroadenoma, adenomatoid tumors or lipoma; lung cancer, in particular small cell lung carcinoma (SCLC), non-small cell lung carcinoma (NSCLC), including squamous cell lung carcinoma, lung adenocarcinoma (LUAD), and large cell lung carcinoma, bronchogenic carcinoma, alveolar carcinoma, bronchiolar carcinoma, bronchial adenoma, lung sarcoma, chondromatous hamartoma and pleural mesothelioma; sarcomas, in particular Askin's tumor, sarcoma botryoides, chondrosarcoma, Ewing's sarcoma, malignant hemangioendothelioma, malignant schwannoma, osteosarcoma and soft tissue sarcomas; soft tissue sarcomas, in particular alveolar soft part sarcoma, angiosarcoma, cystosarcoma phyllodes, dermatofibrosarcoma protuberans, desmoid tumor, desmoplastic small round cell tumor, epithelioid sarcoma, extraskeletal chondrosarcoma, extraskeletal osteosarcoma, fibrosarcoma, gastrointestinal stromal tumor (GIST), hemangiopericytoma, hemangiosarcoma, Kaposi's sarcoma, leiomyosarcoma, liposarcoma, lymphangio sarcoma, lymphosarcoma, malignant peripheral nerve sheath tumor (MPNST), neurofibrosarcoma, plexiform fibrohistiocytic tumor, rhabdomyosarcoma, synovial sarcoma and undifferentiated pleomorphic sarcoma; cardiac cancer, in particular angiosarcoma, fibrosarcoma, rhabdomyosarcoma, liposarcoma, myxoma, rhabdomyoma, fibroma, lipoma and teratoma; bone cancer, in particular osteogenic sarcoma, osteosarcoma, fibrosarcoma, malignant fibrous histiocytoma, chondrosarcoma, Ewing's sarcoma, malignant lymphoma and reticulum cell sarcoma, multiple myeloma, malignant giant cell tumor chordoma, osteochronfroma, osteocartilaginous exostoses, benign chondroma, chondroblastoma, chondromyxoid fibroma, osteoid osteoma and giant cell tumors; hematologic and lymphoid cancer; blood cancer, in particular acute myeloid leukemia, chronic myeloid leukemia, acute lymphoblastic leukemia, chronic lymphocytic leukemia, myeloproliferative diseases, multiple myeloma and myelodysplasia syndrome; Hodgkin's disease; non-Hodgkin's, and the metastases thereof.

17. The pharmaceutical composition or interleukin-2 variant for use according to any one of claims 14 to 16, which is administered to the individual in need thereof in combination with at least one immune check-point inhibitor; preferably anti-PD-1 and/or anti-PDL-1.

18. The pharmaceutical composition according to claim 13, or the interleukin-2 variant according to any one of claims 1 to 9, for use in the prevention or treatment of a disease selected from the group consisting of acute or chronic inflammatory diseases, allergic diseases, autoimmune diseases, graft-versus-host disease and graft rejection.
